# EUROPÄISCHE PATENTSCHRIFT

(11) **EP 1 305 006 B1**
(45) Veröffentlichungstag und Bekanntmachung des Hinweises auf die Patenterteilung: **16.07.2008**
(21) Anmeldenummer: 01969541.0
(22) Anmeldetag: 27.07.2001
(51) Int. Cl.: A61K 9/107, A61K 9/10, A61K 38/13, A61K 31/70

(54) **VERFAHREN ZUR HERSTELLUNG VON DISPERSIONEN ZUR FORMULIERUNG WENIG ODER SCHWER LÖSLICHER WIRKSTOFFE**
PROCESS FOR THE MANUFACTURE OF DISPERSIONS FOR FORMULATING SLIGHTLY OR POORLY SOLUBLE ACTIVE INGREDIENTS
PROCES POUR LA PRODUCTION DES DISPERSIONS POUR LA FORMULATION DE PRINCIPES ACTIFS PEU OU DIFFICILEMENT SOLUBLES

(30) Priorität: 28.07.2000 DE 10036871
(43) Veröffentlichungstag der Anmeldung: 02.05.2003
(73) Patentinhaber: Abbott GmbH & Co. KG, 65205 Wiesbaden (DE)
(72) Erfinder: MÜLLER, Rainer, Helmut, 12161 Berlin (DE)
(74) Vertreter: UEXKÜLL & STOLBERG
(86) Internationale Anmeldenummer: PCT/EP2001/008726
(87) Internationale Veröffentlichungsnummer: WO 2002/009667

(56) Entgegenhaltungen:
- EP-A- 0 296 845
- US-A- 4 707 470
- US-A- 5 529 785
- US-A- 5 534 502
- US-A- 5 622 714
- US-A- 5 660 858
- US-A- 5 662 932
- US-A- 5 916 596

## Beschreibung

Die Erfindung betrifft ein Verfahren zur Herstellung vom Dispersionen, die eine ölige Phase, eine wäßrige Phase und in diesen beiden Phasen wenig löslichen, schwer löslichen bis zu unlöslichen Arzneimittelwirkstoff umfassen.

Wirkstoffe mit geringer Löslichkeit haben sehr oft das Problem einer unzureichenden Bioverfügbarkeit. Der generelle Lösungsansatz für dieses Problem ist die Erhöhung der Löslichkeit dieser Wirkstoffe. Beispiele hierfür sind die Lösungsvermittlung über Solubilisation, Bildung von Einschlußverbindungen (z. B. mit Cyclodextrinen) sowie die Verwendung von Lösungsmittelgemischen (K. H. Bauer, K.-H. Frömming, C. Führer, Pharmazeutische Technologie, Georg Thieme Verlag Stuttgart, 1991). Für viele Wirkstoffe führt dies jedoch nicht zu einer ausreichenden Erhöhung der Löslichkeit, insbesondere wenn Wirkstoffe gleichzeitig schwerlöslich in wäßrigen Medien und gleichzeitig schwerlöslich in organischen Medien sind. Hier scheiden z. B. Lösungsmittelgemische als Lösung für das Problem aus. Alternativ können gering wasserlösliche Wirkstoffe in Ölen gelöst werden, eine O/W-Emulsion hergestellt und diese dann oral oder parenteral (in der Regel i.v.) appliziert werden. Sehr viele Wirkstoffe, insbesondere Wirkstoffe mit gleichzeitig geringer Löslichkeit in wäßrigen und organischen Medien, sind jedoch nicht ausreichend in Ölen löslich. Nicht ausreichend bedeutet, daß aufgrund zu geringer Löslichkeit bei erforderlicher Dosis das zu applizierende Volumen der Emulsion zu groß wird.

In Wasser und in Ölen gering lösliche Wirkstoffe wie Amphotericin B können trotzdem in Emulsionen eingearbeitet werden (Seki et al. US 5 534 502). Um dies zu erreichen müssen jedoch zusätzliche organische Lösungsmittel eingesetzt werden. Diese Lösungsmittel müssen dann in Zwischenschritten der Emulsionsherstellung oder dem Produkt wieder entzogen werden (Davis, Washington, EP 0 296 845 A1) wobei jedoch ein gewisser Restlösungsmittelgehalt im Produkt verbleibt. Zusätzlich ist diese Herstellung sehr zeitaufwendig und kostenintensiv, so daß Produkte basierend auf dieser Technologie praktisch auf dem Markt nicht vertreten sind. Eine alternative Methode ist die Einlagerung von derartigen Substanzen wie Amphotericin B in die Phospholipid-Doppelmembran von Liposomen, Handelsprodukt ist beispielsweise Ambisome^{®} (Janknegt et al., Liposomal and lipid formulations of amphotericin B, Clin. Pharmacokinet., 23, 279-291 [1992]). Nachteilig ist aber auch hier die sehr teure Herstellung, so daß es in der Regel nur in Notfällen eingesetzt wird, wenn eine andere Behandlung nicht zum Ziel führt bzw. nur bei Patienten eingesetzt wird, die finanziell in der Lage sind, die Behandlung zu bezahlen. Somit besteht eindeutig ein Bedarf an einer kostengünstigen Formulierung, die gleichzeitig möglichst einfach herzustellen ist, im Gegensatz zu Liposomen lagerstabil ist und eine Lyophilisation nicht erfordert sowie nicht von Restlösungsmitteln belastet ist.

Der vorliegenden Erfindung liegt daher die Aufgabe zugrunde, ein Verfahren zur Herstellung einer Dispersion zur Verfügung zu stellen, die einen wenig, schwer oder sogar bisher unlöslichen Wirkstoff in einer bisher nicht möglichen Menge gelöst enthält, wobei gleichzeitig die oben beschriebenen Nachteile der Verwendung zusätzlicher zur Formulierung bisher notwendiger organischer Lösungsmittel entfällt.

Gegenstand der vorliegenden Erfindung ist daher ein Verfahren zur Herstellung einer Dispersion auf der Basis einer O/W-Emulsion oder einer W/O-Emulsion beladen mit Wirkstoff, der in Wasser und gleichzeitig auch in Ölen wenig löslich oder schwer löslich bis hin zu unlöslich ist, wobei diese Dispersion frei von toxikologisch bedenklichen organischen Lösungsmitteln ist und den Wirkstoff gelöst in einer Menge enthält, die höher ist als die Menge, die sich additiv aus seiner maximalen Löslichkeit in der Wasser- und der Ölphase der Emulsion ergibt.

Insbesondere ist die erfindungsgemäß gelöste Menge um den Faktor 2, bevorzugter 5, noch bevorzugter 10 oder noch größer als die additive Menge.

Die "additive Menge" wird durch Auflösen der maximalen Wirkstoffmenge in den separaten öligen und wäßrigen Phasen (bei ansonsten identischen Lösebedingungen) entsprechend den Anteilen in der Dispersion ermittelt (Sättigungskonzentrationen), wobei keine weiteren zusätzlichen organischen Lösungsmittel zum Einsatz kommen. Die erfindungsgemäß hergestellte Dispersion enthält zusätzlich zu der additiven Menge ein überadditive Menge an gelöstem Wirkstoff.

Ein wichtiges erfindungsgemäßes Merkmal ist, daß bei gleicher Zusammensetzung hochenergetisch homogenisiert wird, im Vergleich zu niederenergetischem Dispergieren (Schütteln oder Blattrührer).

Die erfindungsgemäße Herstellung der Dispersion erfolgt insbesondere unter Ausschluß von toxikologisch bedenklichen organischen Lösungsmitteln wie z.B. Methylenchlorid und Ethanol. Die Wirkstoffe werden unter Umgehung eines Zwischenschrittes direkt aus der festen Substanz in die Emulsion eingearbeitet.

### Detaillierte Beschreibung der Erfindung

Generell ist es anerkannter Stand der Wissenschaft, daß die Moleküle eines schwerlöslichen oder gering löslichen Wirkstoffes aus dem festen Aggregatzustand (Pulver) über mindestens einen Zwischenschritt (z. B. molekulardisperse Verteilung in einem Lösungsmittel) in eine Emulsion als Trägersystem eingearbeitet werden müssen. Die Erfahrung zeigt, daß bei in Wasser und Öl gleichzeitig sehr gering löslichen Substanzen es nicht genügt, eine Emulsion mit Kristallen des Wirkstoffes zu versetzen. So führt die teilweise praktizierte Zumischung von Amphotericin B-Lösung (Lösungsmittelgemisch) zu einer handelsüblichen O/W-Emulsion wie Intralipid oder Lipofundin zur Präzipitation des Wirkstoffes, es entstehen Amphotericin B-Kristalle, die sedimentieren und sich nicht mehr in der Emulsion auflösen.

Überraschender Weise wurde jedoch nun gefunden, daß die Herstellung eines Emulsionssystems mit gelöstem Wirkstoff auch direkt aus dem festen Aggregatzustand des Wirkstoffes möglich ist. Zur erfindungsgemäßen Herstellung der Dispersion wird der Wirkstoff in partikulärer Form der Wasserphase oder der Ölphase zugesetzt und anschließend alle Komponenten einem höher energetischen oder hochenergetischen Prozeß wie z. B. der Homogenisation, insbesondere der Hochdruckhomogenisation unterzogen. Der hochenergetische Prozeß der Hochdruckhomogenisation führt dazu, daß der Wirkstoff in die Emulsion molekulardispers eingearbeitet wird und keine Wirkstoffkristalle mehr im Polarisationsmikroskop detektierbar sind. Die erhaltenen Emulsionen sind überraschender Weise ähnlich stabil wie Systeme, die unter Einsatz von organischen Lösungsmitteln erzeugt worden sind.

Eine sehr einfache Art der Einarbeitung der Wirkstoffkristalle ist die Verreibung des Wirkstoffes mit einer handelsüblichen O/W-Emulsion (z. B. Lipofundin, Intralipid). Nach Anreiben befindet sich der Wirkstoff primär in der Wasserphase, es ist ein disperses System entstanden, das als innere Phase gleichzeitig Öltropfen und Wirkstoff-Kristalle enthält. Dieses disperse System wird dann homogenisiert oder hochdruckhomogenisiert (z. B. 1.500 bar und 5 - 20 Homogenisationszyklen). Es wird eine feindisperse Emulsion erhalten (Beispiel 1), in der am Ende des Homogenisationsprozesses keine Wirkstoff-Kristalle mehr nachweisbar sind. Die Kristalle haben sich daher nahezu vollständig oder vollständig aufgelöst, d.h. daß sich im Lichtmikroskop selbst bei 1000 facher Vergrößerung in 2 von 3 Feldern nicht mehr als 10 Kristalle, vorzugsweise nicht mehr als 5 Kristalle und insbesondere nicht mehr als 1 Kristall nachweisen lässen/läßt.

Falls es gewünscht ist, kann der Wirkstoff jedoch auch in einer solchen Menge eingesetzt werden, daß am Ende des Homogenisationsprozesses neben dem gelösten Anteil des Wirkstoffs noch ein Anteil des Wirkstoffs in ungelöster kristalliner Form vorliegt, der ein Depot bildet.

Alternativ kann eine wäßrige Suspension des Wirkstoffes mit einer O/W-Emulsion gemischt werden. Es handelt sich wieder um ein disperses System mit einer dispergierten Phase aus Öltropfen und Wirkstoff-Kristallen. Dieses wird ebenfalls einem höher oder hochenergetischem Prozeß wie der Hochdruckhomogenisation unterzogen. Die Zumischung einer wäßrigen Suspension des Wirkstoffes eignet sich insbesondere dann, wenn die Wirkstoffkonzentration relativ gering ist. Zusätzlich kann die wäßrige Suspension des Wirkstoffes vor der Zumischung einem in den Lehrbüchern beschriebenem Mahlprozeß unterzogen werden, z. B. Naßmahlung mit einer Kolloidmühle, einer Kugelmühle oder einer Perlmühle oder durch Hochdruckhomogenisation vorzerkleinert werden.

Generell ist es günstig, den Wirkstoff in der Form sehr feiner Kristalle zu verwenden, d. h. in mikronisierter Form mit einer Teilchengröße im Bereich von ca. 0,1 µm - 25 µm (Kolloidmühle, Gasstrahlmühle).

Alternativ kann der Wirkstoff auch im Öl dispergiert werden. Das Öl mit den Wirkstoff-Kristallen wird dann in der Wasserphase dispergiert, wobei das dafür notwendige Tensid entweder der Wasserphase zugesetzt wird oder in der Ölphase gelöst wird bzw. jeweils dispergiert wird. Im Falle von Lecithin kann das Lecithin im Wasser dispergiert werden oder in der Ölphase unter leichtem Erwärmen gelöst werden.

Bei Einarbeitung der Wirkstoff-Kristalle in die Ölphase kann dies ohne Zusatz eines Tensids erfolgen. Das Tensid, z. B. Lecithin, wird anschließend zugesetzt. Alternativ können auch die Wirkstoff-Kristalle in eine Ölphase eingearbeitet werden, die bereits Tensid enthält.

Nach Einarbeitung der Wirkstoff-Kristalle in das Öl wird die Ölphase in Wasser dispergiert (z. B. mit einem hochtourigen Rührer) und die erhaltene Rohemulsion anschließend hochdruckhomogenisiert. Auch hier ist es günstig, die Wirkstoff-Kristalle möglichst klein einzusetzen. Zur weiteren Zerkleinerung der in die Ölphase eingearbeiteten Wirkstoff-Kristalle kann diese ölige Suspension vor dem Herstellen der Rohemulsion zunächst einer Mahlung unterzogen werden. Die Wirkstoff-Kristalle in der Ölphase werden durch diese Naßmahlung weiter zerkleinert, teilweise bis in den Nanometerbereich. Übliche verfahren der Naßmahlung, die eingesetzt werden können, sind z. B. die Kolloidmühle und die Hochdruckhomogenisation der Ölphase. Generell ist die Kavitation einer wäßrigen Phase das anerkannte Prinzip der Zerkleinerung bei der Hochdruckhomogenisation, d. h. die Anwesenheit von Wasser ist zur Kavitation erforderlich. Öle mit einem zu Wasser extrem geringen. Dampfdruck sind zur Kavitation nicht fähig. Trotzdem wurde überraschender Weise gefunden, daß eine zur Herstellung des neuen Trägersystems ausreichende Zerkleinerung auftritt.

Charakteristisch für die erfindungsgemäße Herstellung der Dispersion ist, daß der in der Emulsion eingearbeitete Wirkstoff in höherer Menge gelöst vorliegt als es sich additiv aus seiner maximalen Löslichkeit in der Wasser- und Ölphase der Emulsion ergibt und gleichzeitig zur Herstellung keine toxikologisch bedenklichen organischen Lösungsmittel eingesetzt wurden. Zu solchen toxikologisch bedenklichen organischen Lösungsmitteln gehören insbesondere Chloroform, Methylenchlorid, längerkettige Alkohole wie Hexanol und Octanol, aber auch Ethanol in höheren Konzentrationen.

In der Regel handelt es sich bei den erfindungsgemäßen Wirkstoffen um Wirkstoffe, die wenig löslich (1 Teil löst sich in 30-100 Teilen Lösungsmittel) oder schwer löslich (1 Teil in 100-1000 Teilen Lösungsmittel), insbesondere aber sehr schwer löslich (1 Teil löst sich in 1.000 bis 10.000 Teilen Lösungsmittel) oder sogar unlöslich sind (> 10.000 Teile Lösungsmittel).

So beträgt die Löslichkeit von Amphotericin B in Wasser weniger als 0,001% (< 0,01 mg/ml) bei pH 6-7, das heißt dem pH-Wert der Emulsion. Die Löslichkeit von Amphotericin ist zwar höher bei pH 2 und pH 11 (0,1 mg/ml), jedoch sind diese Lösungen nicht intravenös applizierbar.

Die Löslichkeit von Amphotericin in Sojaöl (Long Chain Triglycerides - LCT) und in Miglyol 812 (Medium Chain Triglycerides - MCT), den Standardölen für die meisten auf dem Markt befindlichen Emulsionen zur parenteralen Infusion ist kleiner als 0,0001 mg/ml.

40g Emulsion aus Beispiel 1 bestehen zu 20% aus Öl (8g) und ca. 80% aus Wasser (32g). Somit lassen sich aufgrund der Löslichkeiten 8 x 0,0001 mg/ml plus 32 x 0,01 mg, d.h. insgesamt 0,3208 mg Amphotericin in 40g Emulsionsbestandteilen Öl und Wasser auflösen, d.h. 0,008 mg/ml. In der vorliegen erfindungsgemäßen Emulsion konnten 0,2 mg/ml Emulsion eingearbeitet werden (Beispiel 1) ohne daß mikroskopisch Kristalle von ungelöstem Arzneistoff detektierbar waren (Beispiel 12). Auch höhere Konzentration wie 1 mg/ml Emulsion konnten eingearbeitet werden (Beispiel 2), mit Laserdiffraktometrie waren keine der zur Herstellung eingesetzten Arzneistoffpartikel mehr detektierbar (Beispiel 11).

Bei einer gewünschten Dosis von z.B. 100 mg Amphotericin B ergibt sich bei den erfindungsgemäß hergestellten Dispersionen mit 1 bzw. 0,2 mg/ml Emulsion ein intravenös zu applizierendes Volumen von 100 bis 500 ml Emulsion. Somit werden mit der erfindungsgemäß hergestellten Emulsion wenig lösliche und schwer lösliche Wirkstoffe erst in einem ausreichend kleinen Applikationsvolumen bei verträglichen pH-Werten applizierbar.

Gelöster Wirkstoff ist schnell verfügbar. Zur Erzeugung eines Depots kann mehr Wirkstoff in die Dispersion eingearbeitet werden als sich darin löst, d h. man erzeugt Kristalle, die als Depot wirken. Die Löslichkeit in Wasser und Ölphase betragen z.B. für Amphotericin B 0,008 mg/ml, die erfindungsgemäße Emulsion löst ohne detektierbare Kristalle z.B. 0,2 mg/ml (Beispiel 1). Arbeitet man 5 mg/ml Dispersion ein, so ist die Löslichkeit überschritten (übersättigtes System). Nach Hochdruckhomogenisation erhält man zusätzlich zum gelösten Wirkstoff noch hochfeine Arzneistoffkriställchen (Beispiel 15).

Die durch Mischung von Arzneistoff (Beispiel 15) oder einer Arzneistoffsuspension (analog Beispiel 6) mit einer Emulsion und anschließende Homogenisation hergestellten heterogenen, übersättigten Dispersionen sind dadurch gekennzeichnet, daß separat nebeneinander Öltropfen und hochfeine Kriställchen existieren, d.h. die Kristalle sind primär außerhalb der Öltropfen.

Die Bestimmung der Partikelgröße erfolgt mit Lichtmikroskopie unter Ermittlung der Anzahlverteilung. Alternativ erfolgt die Bestimmung mit Laserdiffraktometrie (Gerät: Coulter LS 230, Coulter Electronics, Krefeld, Germany), wobei die erhaltene Volumenverteilung in die Anzahlverteilung umgerechnet wird.

Sind in der Dispersion bei hoher Beladung mit Wirkstoff neben den Emulsionstropfen noch Arzneistoffkristalle vorhanden, so sind direkt nach der Herstellung mindestens 90%, bevorzugt 95% der Anzahl der Wirkstoffkristalle in der Anzahlverteilung kleiner als 5 µm. Bei Anwendung von hohen Drücken (z.B. 1000 bar) und einer ausreichenden Anzahl an Homogenisationszyklen erhält man hochdisperse Systeme. In Abhängigkeit von Druck und Zyklenzahl erhält man Dispersionen mit mindestens 90%, teilweise 95% und insbesondere 99% der Anzahl der Kristalle in der Anzahlverteilung kleiner als 1 µm.

Oben wurde die in situ Erzeugung des Wirkstoff-Depots aus Kriställchen durch erfindungsgemäß Herstellung der Dispersion mit einer Wirkstoffmenge oberhalb der Sättigungslöslichkeit des Systems beschrieben. Alternativ kann auch eine Dispersion erfindungsgemäß mit ausschließlich gelöstem Wirkstoff hergestellt werden, der man nachträglich Wirkstoffkristalle definierter Größe zumischt, z.B. mikronisierter Wirkstoff.

Zur erfindungsgemäßen Herstellung der Dispersion können handelsübliche O/W-Emulsionen eingesetzt werden (z.B. Lipofundin, Intralipid, Lipovenös, Abbolipid, Deltalipid und Salvilipid) oder es wird eine Emulsion aus Ölphase, Emulgator / Stabilisator und äußerer Phase (z.B. Wasser) hergestellt.

Beispiele für Bestandteile der Ölphase der Emulsionen sind: Sojaöl, Safloröl (Distelöl), langkettige Triglyceride (LCT), mittelkettige Triglyceride (MCT) wie z.B. Miglyole, Fischöle und Öle mit einem erhöhten Anteil an ungesättigten Fettsäuren, acetylierte Partialglyceride wie Stesolid, einzeln oder in Mischungen.

Zur Stabilisierung der Dispersionen können Emulgatoren und Stabilisatoren eingesetzt werden. Diese sind gegebenenfalls bereits in der zur erfindungsgemäßen Herstellung der Dispersion eingesetzten Emulsion enthalten, Zusatz weiterer Emulgatoren und Stabilisatoren bei der Herstellung der Dispersion kann vorteilhaft sein.

Beispiele für Emulgatoren sind z.B. Ei-Lecithin, Soja-Lecithin, Phospholipide aus Ei oder Soja, Tween 80, Natriumglykocholat und Natriumlaurylsulfat (SDS). Alternativ kann Stabilisierung durch Zusatz von Substanzen erfolgen die über andere Mechanismen als Emulgatoren stabilitätserhöhend wirken, z.B. über sterische Stabilisierung oder Erhöhung der Zetapotentials. Solche Stabilisatoren sind z.B. Block-Copolymere wie z.B. Poloxamere (z.B. Poloxamer 188 und 407) und Poloxamine (z.B. Poloxamine 908), Polyvinylpyrrolidon (PVP), Polyvinylalkohol (PVA), Gelatine, Polysaccharide wie Hyaluronsäure und Chitosan und ihre Derivate, Polyacrylsäure und ihre Derivate, Polycarbophil, Cellulosederivate (Methyl-, Hydroxypropyl- und Carboxymethylcellulose), Zuckerester wie Saccharosemonostearat und Antiflokkulatien wie Natriumcitrat. Emulgatoren und Stabilisatoren können einzeln oder in Mischungen verwendet werden. Typische Konzentrationen sind 0,1% bis 20%, insbesondere 0,5% bis 10%.

Als wäßrige äußere Phase der zur erfindungsgemäßen Herstellung der Dispersion eingesetzten O/W-Emulsion können dienen: Wasser, Mischungen von Wasser mit anderen wassermischbaren organischen Flüssigkeiten, flüssige Polyethylenglykole (PEG, insbesondere PEG 400 und 600).

Die wäßrige äußere Phase kann auch Zusätze enthalten, z.B. Elektrolyte, Nichtelektrolyte (z.B. Glycerol, Glucose, Mannit, Xylit zur Isotonisierung), Gelbildner wie Cellulosederivate und Polysaccharide wie Xanthan und Alginate (z.B. zur Viskositätserhöhung).

Für die topische Applikation können der Dispersion Penetrationsverstärker (z.B. Azone, Laurinsäure) und für die Applikation zum Gastrointestinaltrakt Absorptionsverstärker (z.B. Gallensäuren, Lysophospholipide) zugesetzt werden.

Wirkstoffe zur Einarbeitung in die Emulsion sind neben Amphotericin B z.B. Ciclosporin, Buparvaquon und Atovaquon. Weitere Wirkstoffe sind Hormone (z.B., Estradiol), Antioestrogene und Kortikoide (z.B. Prednicarbat).

Die Applikation der Emulsion kann auf verschiedenen Wegen erfolgen, z.B. parenteral aber auch oral oder topisch. Bei parenteraler Applikation sind alle gängigen Wege möglich; z.B. intravenös, intra- und subkutan, intramuskulär, intraartikulär, intraperitoneal etc.

Topische Emulsionen mit Ciclosporin können die Wirkstoffpenetration in die Haut verbessern aufgrund des hohen gelösten Anteils an Arzneistoff (erhöhter Konzentrationsgradient). Orale Applikation der Ciclosporin-Emulsion kann die Bioverfügbarkeit erhöhen da im Gegensatz zu mikronisiertem Ciclosporin ein erhöhter gelöster Anteil vorliegt.

Die Bioverfügbarkeit von oral appliziertem Amphotericin B ist aufgrund seiner geringen Löslichkeit nahezu Null. Orale Applikation der Amphotericin-Emulsion kann aufgrund des erhöhten gelösten Anteils ebenfalls die Bioverfügbarkeit erhöhen.

Die erfindungsgemäß hergestellten Emulsionen (z.B. mit Buparvaquon und Atovaquon) können nach intravenöser Injektion auch durch Anlagerung einer Targeting-Einheit (z.B. Apolipoprotein E in Kombination mit Apolipoprotein AI und AIV) für eine gewebsspezifische Arzneistoffapplikation eingesetzt werden (Targeting zum Gehirn). Erreger lokalisieren bei bestimmten Erkrankungen des monozytären phagocytierenden Systems (MPS) auch im Gehirn und sind bisher schwer einer Therapie zugänglich (z.B. Leishmaniosen, Toxoplasmose).

Die oben beschriebenen Systeme sind vom Typ O/W, d. h. Öltropfen sind dispergiert in einer Wasserphase. Es ist jedoch auch möglich, Dispersionen auf der Basis von W/O-Emulsionen zu produzieren. Ein grundsätzlicher Vorteil ist, daß die äußere Ölphase als eine Diffusionsbarriere fungiert und die Freigabe des Arzneistoffes verzögert. Derartige Dispersionen können nicht intravenös appliziert werden, aber sie können zum Beispiel intramuskulär oder subkutan als Depotformulierung injiziert werden. Applikation dieser W/O-Systeme am Auge erhöht die Verweilzeit aufgrund der erhöhten Viskosität und gleichzeitig wird eine verlängerte Arzneistofffreisetzung erreicht. Bei topischer Applikation auf die Haut hat die Ölphase einen okklusiven Effekt, der zu einer erhöhten Arzneistoffpenetration führt. Daher besitzen diese W/O-Typ-Systeme einen Vorteil für spezielle Anwendungen. Bevorzugte Form der Erfindung ist jedoch die Dispersion auf der Basis des O/W-Typs.

Bei Öl-in-Wasser Emulsionen ist die Dispersion dadurch gekennzeichnet, daß sie 5 bis 99,5 Gew.-% wäßrige Phase, vorzugsweise 10 bis 95 Gew.-% wäßrige Phase, besonders bevorzugt 60 bis 95 Gew.-% wäßrige Phase und speziell 70-95% wäßrige Phase, jeweils bezogen auf die Gesamtmenge der Dispersion, enthält.

Bei Wasser-in-Öl Emulsionen ist die Dispersion dadurch gekennzeichnet, daß sie aus 5 bis 30 Gew.-% wäßriger Phase, vorzugsweise 10 bis 25 Gew.-% wäßriger Phase, besonders bevorzugt 10 bis 20 Gew.-% wäßriger Phase, jeweils bezogen auf die Gesamtmenge der Dispersion, enthält.

Die Bestandteile der Ölphase der Emulsionen sind - wie oben ausgeführt - insbesondere ausgewählt aus der Gruppe bestehend aus Sojaöl, Safloröl (Distelöl), langkettigen Triglyceriden (LCT), mittelkettigen Triglyceriden (MCT), wie z. B. Miglyole, Fischölen und Ölen mit einem erhöhten Anteil an ungesättigten Fettsäuren, acetylierten Partialglyceriden, wie in Stesolid®, einzeln oder in Mischungen. Die mittelkettigen Triglyceride enthalten vorzugsweise wenigstens 90 % Triglyceride der Capryl-Säure (C8) und der Caprin-Säure (C10). Als Ölphase sind im Rahmen der Erfindung Gemische aus Sojaöl und MCT, vorzugsweise im Gewichtsverhältnis 5:1 bis 1:5, besonders bevorzugt zwischen 2:1 und 1:2 oder 1:1 geeignet.

Die Fettphase der Dispersion kann aus Ölen bestehen, d.h. die Lipide sind bei einer Raumtemperatur von 20°C flüssig. Es besteht weiterhin die Möglichkeit, daß diese Öle mit Lipiden gemischt werden, die bei einer Raumtemperatur von 20°C fest sind.

Die Mischungsanteile von Öl zu festem Lipid können von 99 + 1 bis 1 + 99 (Gewichtsanteile) variieren. Bevorzugt sind Mischungen, die mindestens 10 Teile flüssiges Öl enthalten, speziell mindestens 30 Teile flüssiges Öl und insbesondere mindestens 50 Anteile flüssiges Öl.

In speziellen Fällen kann die Lipidphase der Dispersion zu 100% Lipide enthalten, die bei einer Raumtemperatur von 20°C fest sind. Schmelzen die Lipide nahe der Raumtemperatur, können Dispersionen erhalten werden, deren Lipidtröpfchen sich in einem Zustand einer "Unterkühlten Schmelze" befinden. Liegen sehr hochschmelzende Lipide vor, können - ungeachtet der durch die Thomson-Gleichung beschriebenen Schmelzpunktionsdepression - die Partikel der Dispersion aushärten. Die Thomson-Gleichung beschreibt, daß der Schmelzpunkt von Lipiden gegenüber ihrer "bulk"-Ware stark herabgesetzt wird, wenn diese in sehr feinen Partikeln auskristallisieren (z. B. Nanopartikel oder Partikel in einem Größenbereich von wenigen Mikrometern) (Hunter, R.J., Foundations of colloid science, Vol. 1, Oxford University Press, Oxford, 1986).

Beispiele für bei Raumtemperatur feste Lipide sind, Karnaubawachs, Hydroxyoctacosanylhydroxystearat, Chinesisches Wachs, Cetylpalmitat, Bienenwachs und ähnliche Wachse. Weitere Beispiele für feste Lipide beinhalten C₂₀₋₄₀ Di- und Triglyceride, mit gesättigten und ungesättigten Fettsäuren, C₂₀₋₄₀ Fettalkohole, C₂₀₋₄₀ Fettamine und ihre Verbindungen, sowie Sterole.

Als Lipide zur Herstellung von Mischungen aus flüssigen und festen Lipiden sind geeignet: Natürliche oder synthetische Triglyceride bzw. Mischungen derselben, Monoglyceride und Diglyceride, alleine oder Mischungen derselben oder mit z. B. Triglyceriden, selbst-emulgierende modifizierte Lipide, natürliche und synthetische Wachse, Fettalkohole, einschließlich ihrer Ester und Ether und Mischungen derselben. Besonders geeignet sind synthetische Monoglyceride, Diglyceride und Triglyceride als individuelle Substanzen oder als Mischung (z. B. Hartfett), Imwitor 900, Triglyceride (z. B. Glyceroltrilaurat, Glyceroltrimyristat, Glyceroltripalmitat, Glyceroltristearat und Glyceroltribehenat) und Wachse wie z. B. Cetylpalmitat, Karnaubawachs und weißes Wachs (DAB). Außerdem Kohlenwasserstoffe, wie z. B. Hartparaffin.

Die Tropfengröße der Öltropfen (O/W-Typ) oder Wassertropfen (W/O-Typ) in der Dispersion ist größer als 100 nm (bestimmt mit Photonenkorrelationsspektroskopie - PCS). Das empfohlene obere Größenlimit für die Tropfen ist 10 µm, anderenfalls kommt es zum Aufrahmen aufgrund der Flotation der Tropfen, was zu physikalischer Instabilität führt (Tropfenkoaleszenz). Um Flotation zu minimieren, sollte die Größe kleiner als 5 µm sein, vorzugsweise unterhalb von 1 µm (PCS-Durchmesser), was zu den sogenannten physikalisch "autostabilen" Dispersionen führt. Die optimale Stabilität wurde gefunden im Größenbereich ähnlich zu parenteralen Fettemulsionen mit PCS-Durchmessern von 200 nm bis 500 nm.

Der Gehalt an Stabilisatoren in parenteralen Zubereitungen sollte so niedrig wie möglich gehalten werden, um Toxizität und Störungen des Metabolismus zu minimieren. Von Lecithin-haltigen Emulsionen zur parenteralen Ernährung ist es bekannt, daß eine zu hohe Zuführung von Lecithin metabolische Störungen bewirken kann, typische Tagesvolumina appliziert sind hier z. B. 500 ml Emulsion und mehr. Dies führte zu der Entwicklung der Lecithinreduzierten Emulsionen, d. h. man reduzierte den Lecithingehalt von 1,2% weiter auf nur 0,6% Lecithin. Einige Systeme zur Applikation von schwerlöslichen Arzneistoffen verwenden einen relativ hohen Emulgatorgehalt (z. B. Solubilisierung mit Tensiden, SEDDS - self-emulsifying drug delivery systems basierend auf der Solubilisation von Öl mit hohen Tensidkonzentrationen). Eine spezielle Eigenschaft der vorliegenden Erfindung ist, daß sie die Tensidbelastung minimiert. Eine typische Zusammensetzung des O/W-Types der erfindungsgemäß hergestellten Dispersion ist: 20 g Öl, 1,2 g Lecithin, 0,1 g Arzneistoff und 78,3 g Wasser. Dies bedeutet, daß die 21,2 g produzierter Öltropfen aus 20 g Ölphase (= 94,3%) und 1,2 g Stabilisator (= 5,7%) bestehen.

Weitere Beispiele für Emulgatoren sind neben Lecithinen die Polyethoxysorbitanester (Tween'-Typen), wie beispielsweise Laurate (Tween 20/21), Palmitate (Tween 40), Stearate (Tween 60/61), Tristearate (Tween 65), Oleate (Tween 80/81), oder Trioleate (Tween 85), Natriumglycocholat und Natriumlaurylsulfat (SDS) sowie die Sorbitanfettsäureester (Span^{®}-Typen). Besonders bevorzugt ist Tween 80.

Bevorzugt werden weiterhin Tenside, Emulgatoren und Stabilisatoren eingesetzt, die für die Anwendung am und im Menschen zugelassen sind (z.B. Hilfsstoffe mit dem GRAS-Status).

Speziell für die Dispersionen vom Typ W/O werden die typischen Wasser-in-Öl-Tenside zur Stabilisierung benutzt, manchmal in Mischungen, auch in Mischungen mit O/W-Emulgatoren. Beispiele hierfür sind die Fettalkohole, Ethylenglykolmonostearat, Glycerolmonostearat, Sorbitanfettsäureester (span^{®}-Serie, z. B. Span 20-, Span 40-, Span 60- und Span 80-Serie, speziell Span 85), Ether von Fettalkoholen mit Polyethylenglykol (PEG) (z. B. Brij^{®}-Serie), Ester von Fettsäuren mit PEG (z. B. Myrj^{®}-Serie).

Im allgemeinen werden wieder Tenside und Stabilisatoren mit einem anerkannten Status bevorzugt, z. B. GRAS-Substanzen (Generally Regarded As Safe - Food Additives - GRAS substances, Food Drug Cosmetic Law Reports, Chicago (1994), Food Additive Database der FDA, Internet: www.fda.gov, 1999).

Im Fall daß die erfindungsgemäß hergestellten Dispersionen - zusätzlich zu den Öltropfen - noch Partikel von ungelöstem Wirkstoff enthalten, sollte die Partikelgröße so klein wie möglich sein, zum Beispiel zwecks Erhalt der physikalischen Stabilität und zur Vermeidung von Sedimentation. Zusätzlich, im Fall der intravenösen Applikation, sollten die Partikel klein genug sein, um Kapillarblockade zu vermeiden. Die kleinsten Blutkapillaren sind ungefähr 5-6 µm im Durchmesser. Daher sollte der Partikeldurchmesser 90% unterhalb von 5 µm sein, vorzugsweise auch der Durchmesser 95% und insbesondere der Durchmesser 100% sollte unterhalb 5 µm sein (gemessen mit Laserdiffraktometrie nach Abtrennung der Partikel von der Dispersion durch Zentrifugation, Volumenverteilungsdaten). Es ist noch günstiger, wenn diese Durchmesser alle unterhalb von 3 µm sind, da dann eine Sicherheitsdistanz zur Größe der kleinsten Kapillaren vorhanden ist.

Am vorteilhaftesten ist eine Partikelgröße des ungelösten Arzneistoffes unterhalb von 1000 nm (mittlere Partikelgröße gemessen mit Photonenkorrelationsspektroskopie). Diese Größe ist weit weg von den 5-6 µm der kleinsten Kapillardurchmesser und schließt gleichzeitig jegliche Sedimentationseffekte aus (diese Partikelgröße sedimentiert nicht relativ unabhängig von der Dichte des Arzneistoffes). Im Fall, daß eine schnellere Auflösung der Arzneistoffkristalle nach Applikation der Dispersion notwendig ist, sollte der mittlere PCS-Durchmesser im Bereich 100 nm bis ungefähr 400 nm, bevorzugt unter 100 nm sein.

Generell ist es günstig, den Wirkstoff zur Herstellung der Dispersion in der Form sehr feiner Kristalle zu verwenden, d.h., in mikronisierter Form mit einer mittleren Teilchengröße im Bereich von ca. 0,1 µm - 25 µm (Kolloidmühle, Gasstrahlmühle). Bevorzugt sind mittlere Teilchengrößen von 0,1µm - 5 µm, besonders bevorzugt von kleiner als 1 µm.

Der pH-Wert der erfindungsgemäß hergestellten Dispersionen liegt typischerweise zwischen 4 und 8, vorzugsweise zwischen 5 und 7,5, besonders bevorzugt zwischen 6 und 7,5 und wird in der Praxis bestimmt durch die Applikationsform.

Die Dispersion kann ferner eine wirksame Menge eines Antioxidanz, wie beispielsweise Vitamin E, insbesondere das Isomer alpha-Tocopherol enthalten. Alternativ können auch beta- oder gamma-Tocopherol, oder Ascorbylpalmitat verwendet werden. Der Zusatz kann zwischen 10 mg und 2000 mg, vorzugsweise zwischen 25 mg und 1000 mg, bezogen auf 100 g Triglyceride betragen.

Eine typische gemäß der Erfindung hergestellte Dispersion kann somit, bezogen auf die anwendungsfertige Gesamtzusammensetzung z.B. umfassen: 0,05 bis 1,0 Gew.-%, vorzugsweise 0,05 bis 0,5 Gew.-% des Wirkstoffes, 0,05 bis 2 Gew.-% eines Emulgators oder Emulgatorgemisches, beispielsweise Tween 80 und/oder Ei-Lecithin, dispergiert in einer O/W-Emulsion, die, bezogen auf die Emulsion, 5 bis 30 Gew.-%, vorzugsweise 10 bis 20 Gew.-% Triglyceride enthält. Bei den Triglyceriden handelt es sich vorzugsweise um Sojabohnenöl, mittelkettige Triglyceride (wenigstens 90 % C8/C10) sowie Gemische aus Sojabohnenöl und mittelkettigen Triglyceriden (wenigstens 90 % C8/C10) im Gewichtsverhältnis 1:2 bis 2:1, vorzugsweise 1:1. Daneben können noch, bezogen auf die Gesamtzusammensetzung, 0,5 bis 5 Gew.-%, vorzugsweise 1 bis 3 Gew.-% übliche Isotonisierungsmittel, wie Glycerol, und 0,005 bis 0,05 Gew.-% Antioxidantien, wie beispielsweise alpha-Tocopherol enthalten sein. Ein besonders bevorzugter Wirkstoff ist insbesondere Amphotericin B. Zusätzlich können auch Konservierungsmittel zugesetzt werden. Die trifft insbesondere bei Abpackung der Dispersion in Gefäße zur Mehrfachentnahme zu.

Die Dispersion enthält den Wirkstoff gelöst in einer Menge, die größer ist als die Menge, die sich additiv aus seiner maximalen Löslichkeit jeweils in der Wasser- und der Ölphase der Emulsion ergibt, wobei die "additive Menge" unter Normalbedingungen (20°C, Normaldruck) durch Auflösen der maximalen Wirkstoffmenge in den separaten öligen und wäßrigen Phasen (bei ansonsten identischen Lösebedingungen) entsprechend den Anteilen in der Dispersion ermittelt (Sättigungskonzentrationen) wird.

In der Dispersion sind typische Wirkstoffkonzentrationen 0,01 Gew.-% bis 30 Gew.-%, vorzugsweise 0,1 Gew.-% bis 10 Gew.-%, besonders bevorzugt 1 Gew.-% bis 5 Gew.-%, bezogen auf die Gesamtmenge der Dispersion.

Arzneistoffe von besonderem Interesse - neben Amphotericin B - sind Vancomycin und Vecuronium. Des weiteren können schwerlösliche Arzneistoffe aus den Gruppen der Prostaglandine, z. B. Prostaglandin E₂, Prostaglandin F₂ₐ und Prostaglandin E₁, Proteinase-Hemmstoffe, wie z. B. Indinavir, Nelfinavir, Ritonavir, Saquinavir, Zytostatika, z. B. Paclitaxel, Doxorubicin, Daunorubicin, Epirubicin, Idarubicin, Zorubicin, Mitoxantron, Amsacrin, Vinblastin, Vincristin, Vindesin, Dactiomycin, Bleomycin, Metallocene, z B. Titanmetallocendichlorid, und Lipid-Arzneistoff-Konjugate, wie z. B. Diminazenstearat und Diminazenoleat, und generell schwerlösliche Antiinfektiva wie Griseofulvin, Ketoconazol, Fluconazol, Itraconazol, Clindamycin, insbesondere antiparasitische Arzneistoffe, z. B. Chloroquin, Mefloquin, Primaquin, Pentamidin, Metronidazol, Nimorazol, Tinidazol, Atovaquon, Buparvaquon, Nifurtimox und antiinflammatorische Arzneistoffe, wie z. B.Ciclosporin, Methotrexat, Azathioprin, verwendet werden.

Dispersionen, die antiinflammatorische Arzneistoffe enthalten, können topisch, oral und parenteral angewendet werden. Im Falle einer topischen Anwendung auf der Haut, kann der Arzneistoff in das tiefere Gewebe penetrieren, wo entzündliche Prozesse stattfinden. Mit einer topischen Anwendung auf Schleimhäuten, wie z. B. am Auge, können Erkrankungen wie das "Trockene Auge"-Syndrom behandelt werden, dem ein entzündlicher Prozeß zugrunde liegt. Eine topische Anwendung auf den Schleimhäuten der Vagina ist ebenso vorteilhaft, z. B. ganz besonders für Antiinfektiva. Die Dispersion spreitet gut auf der Schleimhautoberfläche und gewährleistet so eine gleichmäßige Verteilung des Arzneistoffs. Insbesondere wenn diese Dispersion Öltröpfchen und zusätzlich sehr feine Arzneistoffkristalle enthält, da diese feinen Kristalle auf der vaginalen Schleimhaut haften und sich dort langsam auflösen und damit für eine verlängerte Arzneistoffwirkung sorgen (Depotwirkung). Für eine Anwendung am Auge ist es vorteilhaft, wenn man Dispersionen verwendet, die positiv geladen sind. Die Wechselwirkungen der positiv geladenen Partikel mit den negativ geladenen Zellmembranen verlängern die Verweilzeit des Arzneistoffs am Wirkort.

Die orale Anwendung der Dispersion ist geeignet, die Bioverfügbarkeit von schwerlöslichen Arzneistoffen, die oral nicht ausreichend verfügbar sind, zu erhöhen. Beispiele hierfür sind Paclitaxel und Amphotericin B. Anstelle von wäßrigen Dispersionen können auch, durch Sprühtrocknung oder Gefriertrocknung überführte, trockene Formen verwendet werden.

Die parenterale, insbesondere die intravenöse Anwendung von arzneistoffhaltigen Dispersionen kann Nebenwirkungen reduzieren, z. B. bei Doxorubicin, Daunorubicin und Amphotericin B. Intravenös angewendete Dispersionen können durch Modifizierung der Oberfläche mit Apolipoproteinen gezielt zu gewünschten Zielorganen, wie Gehirn oder Knochenmark gelenkt werden. Dies ist bei Arzneistoffen, die keinen oder nur geringen Zugang zum Gehirn haben, von besonderem Interesse. Typische Beispiele hierfür sind zytotoxische Substanzen wie Doxrubicin. Eine gezielte Aufnahme zytotoxischer Dispersionen in das Gehirn ermöglicht die Behandlung von Hirntumoren, die bisher nur operativ oder lokal, z. B. mit implantierten therapeutischen Systemen und mit arzneistoffhaltigen Implantaten behandelt werden können. Dispersionen, die Antiinfektiva mit geringer Blut-Hirn-Schranken-Permeabilität enthalten, können nun genutzt werden, um diese Antiinfektiva zur Behandlung von persistierenden Parasiten durch die Blut-Hirn-Schranke zu transportieren.

Die Organverteilung von intravenös applizierten Arzneistoffträgern wird von deren physiko - chemischen Eigenschaften, wie z. B. Partikelgröße, Partikelladung und Oberflächenhydrophobie bestimmt. Negativ geladene Partikel werden zum Beispiel wesentlich schneller von den Makrophagen der Leber aufgenommen als ungeladene Partikel (Wilkens, D, J. and Myers, P. A., Studies on the relationship between the electrophoretic properties of colloids and their blood clearance and organ distribution in the rat. Brit. J. Exp. Path. 47, 568-576, 1966). Um die In-vivo-Organverteilung zu modifizieren, kann die Ladung der erfindungsgemäß hergestellten Dispersion geändert werden, speziell positiv geladene Dispersionen sind vorteilhaft. Die positiv geladene Dispersion kann im Bereich der Einstichstelle an den negativ geladenen Zelloberflächen haften bleiben. Nach intravenöser Applikation der negativ geladenen Dispersion interagieren die Partikel mit negativ geladenen Proteinen, speziell mit Albumin, das mengenmäßig bedeutendste Protein im Blut. Aufgrund seiner Funktion als Dysopsonin kann es durch Adsorption an der Tropfenoberfläche und Bildung einer Albumin-Adsorptionsschicht die Verweilzeit der erfundenen Dispersion im Blut verlängern (z. B. verminderte Aufnahme durch Makrophagen der Leber).

Positiv geladene Dispersionen können unter Verwendung positiv geladener Emulgatoren, Mischungen von positiv geladenen und ungeladenen Stabilisatoren (z. B. Poloxamere) und/oder negativ geladenen Emulgatoren (z. B. Lecithin) hergestellt werden. Positiv geladene Dispersionen haben ein positives Zetapotential. Das Zetapotential der Dispersionspartikel wird mit elektrophoretischer Messung in destilliertem Wasser (durch Zugabe von Natriumchlorid auf eine Leitfähigkeit von 50 µS/cm eingestellt) oder im Originaldispersionsmedium (äußere Phase der Dispersion) gemessen. Beispiele für positiv geladene Emulgatoren und Stabilisatoren sind Stearylamin, Cetypyridiniumchlorid (CPC), für positiv geladene Lipide N-[1-(2,3-dioleyloxy)propyl]-N,N,N-trimethylammoniumchlorid (DOTMA), Didodecyldimethylammoniumbromid (DDAB), 2,3-Dioleyloxy-N-[2(spermidincarboxamid)ethyl]-N,N-dimethyl-1-propylammonium-trifluoroacetat (DOSPA), 3β-[N-(N',N'-Dimethylaminoethan)carbamoyl]-cholesterol (DC-Chol).

Die Herstellung positiv geladener Dispersionen kann unter Verwendung positiv geladener Emulgatoren oder Emulgatormischungen im Produktionsprozeß durchgeführt werden (De novo-Herstellung). Der positiv geladene Emulgator kann alternativ auch zu einer negativ geladenen Dispersion zugefügt werden. Der Emulgator muß in ausreichender Menge zugefügt werden, damit eine Ladungsumkehr von negativ nach positiv eintritt.

Nähere Beschreibung des Produktionsprozesses: Die Mischung aus Lipid, Arzneistoff, Wasser und Emulgator oder andere Stabilisatoren muß einem hochenergetischem Dispergierprozeß unterzogen werden. Sollen Mischungen von Ölen und festen Fetten im Homogenisationsansatz verwendet werden, ist es vorteilhaft, das feste Fett bei erhöhter Temperatur im Öl zu lösen. Die bevorzugte erfindungsgemäße Methode die Dispersion herzustellen, ist die Hochdruckhomogenisation, z. B. mit Kolben-Spalt-Homogenisatoren oder Jet Stream-Homogenisatoren. Befindet sich Wasser in der äußeren Phase der Dispersion, wird die Homogenisation zwischen 0°C und 100°C durchgeführt. Die beste Dispergierung und schnellste Auflösung des schwerlöslichen Arzneistoffs wird erreicht, wenn die Homogenisation deutlich über Raumtemperatur durchgeführt wird, z. B. zwischen 35°C und 100°C. Die optimale Homogenisationstemperatur bei gleichzeitiger Berücksichtigung der chemischen Stabilität des Arzneistoffs wurde zwischen 45°C und 65°C ermittelt. Liegt ein extrem temperaturempfindlicher Arzneistoff vor, sollte die Homogenisation in der Nähe des Gefrierpunktes von Wasser durchgeführt werden (z. B. ungefähr 4°C).

Werden für die äußere Phase der Dispersion andere Flüssigkeiten als Wasser verwendet, die einen höheren Siedepunkt als Wasser besitzen, kann auch bei höheren Temperaturen oder unter 0°C (z. B. PEG 600) homogenisiert werden.

Im Fall von Mischungen aus Lipiden, Mischen von Öl und festem Lipid als "bulk"-Waren kann zu einer festen "bulk"-Mischung führen - obwohl die daraus in der Dispersion produzierten Partikel flüssig sind (Thomson-Effekt). In diesem Falle sollte die Homogenisation bei einer Temperatur durchgeführt werden, die über dem Schmelzpunkt der "bulk"-Mischung liegt. Dasselbe gilt bei alleiniger Verwendung von festen Lipiden zur erfindungsgemäßen Herstellung der Dispersion. Der angelegte Homogenisationsdruck kann zwischen 10 und 11.000 bar liegen. Werden die Dispersionen mit 11.000 bar produziert, ist die resultierende Dispersion steril, da unter diesem hohen Druck Bakterien und Viren zerrissen werden. Ist eine Sterilisation durch Homogenisation nicht erwünscht, liegt der bevorzugte Produktionsdruck zwischen 200 bar und annähernd 4000 bar. Die in der Industrie in Produktionslinien verwendeten Hochdruckhomogenisatoren arbeiten gewöhnlich in einem Bereich von 200 bar bis 700 bar, daher wäre es nicht notwendig neue Maschinen anzuschaffen, wenn bei diesen Drücken gearbeitet wird. Die Produktion bei niedrigeren Drücken erfordert jedoch eine höhere Anzahl an Durchläufen (Zyklen). Muß eine höhere Anzahl an Durchläufen vermieden werden (z. B. begründet durch Aspekte der chemischen Stabilität des Arzneistoffes), sollte ein höherer Druck angewendet werden, der von 700 bar bis 4000 bar reicht. Für den Bereich 700-1500 bar können Homogenisatoren von APV Gaulin (Lübeck, Deutschland) verwendet werden, für den Bereich 700-2000 bar sind Maschinen der Firma Niro Soavi (Lübeck, Germany) geeignet, des weiteren ermöglichen spezielle Homogenisatoren der Firma Stansted (Stansted, UK) bei Drücken bis zu 4000 bar zu arbeiten.

Um die Dispersion herzustellen kann jede Homogenisatorausstattung verwendet werden, die eine genügend hohe Leistungsdichte erreicht, d. h. typischerweise über 10⁴ W/m³. Bei einigen Homogenisatoren kann die Leistungsdichte (dissipierte Energie pro Volumeneinheit der Dispergierzone) nicht errechnet werden, da die genaue Größe der Dispergierzone nicht bekannt ist (z. B. Microfluidizer). In diesem Fall muß die Eignung der Maschine für die Herstellung der Dispersion auf empirischem Wege ermittelt werden. Beispiele für Homogenisatoren vom Kolben-Spalt-Typ sind die Maschinen von den Firmen APV Gaulin, Niro Soavi, Stansted und French Press, ein Beispiel für Jet Stream-Homogenisatoren ist der Microfluidizer (Microfluidics, Inc., USA).

Die Erfindung wird durch die nachfolgenden Beispiele näher erläutert, ohne sie jedoch zu beschränken.

### Beispiele

### Beispiel 1

8 mg Amphotericin B wurden mit 40 g Lipofundin N 20 % angerieben (0,2 mg Amphotericin B/ml Emulsion) und die erhaltene Dispersion mit einem Ultra-Turrax-Rührer 5 Minuten bei 8000 Umdrehungen pro Minute gerührt. Anschließend wurde die Dispersion mit einem Micron LAB 40 bei 1.500 bar mit 20 Zyklen hochdruckhomogenisiert. Die Partikelgröße wurde mit einem Laserdiffraktometer bestimmt (Coulter LS 230, Coulter Electronics, USA). Der Durchmesser 50 % (D50%) der Volumenverteilung betrug 0,164 µm, D90% 0,340 µm, D95% 0,387 µm, D99% 0,466 µm und D100% 0,700 µm.

### Beispiel 2

Es wurde ein Emulsionssystem mit Amphotericin B wie in Beispiel 1 hergestellt, die eingearbeitete Menge an Amphotericin B betrug jedoch 40 mg (d. h. 1 mg/ml Emulsion). Es wurden folgende Durchmesser gemessen: D50% 0,160 µm, D90% 0,362 µm, D95% 0,406 µm, D99% 0,485 µm und D100% 0,746 µm.

### Beispiel 3

Es wurde eine Emulsion analog Beispiel 1 hergestellt, die eingearbeitete Amphotericin B-Menge betrug jedoch 80 mg (d. h. 2 mg/ml Emulsion). Es wurden folgende Durchmesser gemessen: D50% 0,194 µm, D90% 0, 381 µm, D95% 0,423 µm, D99% 0,494 µm und D100% 0,721 µm.

### Beispiel 4

40 mg Amphotericin B-Pulver wurden mit 40 g Öl (Mischung 50 : 50 aus LCT und MCT) angerieben und die erhaltene Suspension wie in Beispiel 1 mit einem Ultra-Turrax für 5 Minuten gerührt. Anschließend wurde die Suspension mit einem Hochdruckhomogenisator Micron LAB 40 hochdruckhomogenisiert mit 2 Zyklen bei 150 bar, 2 Zyklen bei 500 bar und anschließend 20 Zyklen bei 1.500 bar. 8 g der erhaltenen öligen Suspension wurden dann in 32 g Wasser dispergiert, das 1,2 % Lecithin enthielt. Dispergierung erfolgte mit einem Ultra-Turrax für 5 Minuten bei 8000 Umdrehungen/Minute. Die erhaltene Dispersion wurde dann mit dem Micron LAB 40 hochdruckhomogenisiert bei 500 bar mit 10 Zyklen. Es wurden folgende Durchmesser gemessen: D50% 0,869 µm, D90% 2,151 µm, D95% 2,697 µm, D99% 3,361 µm.

### Beispiel 5

Es wurde eine Emulsion analog Beispiel 4 hergestellt, allerdings erfolgte die Herstellung der Emulsion mit Hochdruckhomogenisation nicht bei Raumtemperatur, sondern in einem temperaturkontrolliertem LAB 40 bei 50°C. Es wurden folgende Durchmesser gemessen: D50% 0,647 µm, D90% 1, 537 µm, D95% 1,768 µm, D99% 2,152 µm und D100% 3, 310 µm.

### Beispiel 6

Es wurde eine Amphotericin B-Emulsion durch Hochdruckhomogenisation analog Beispiel 1 hergestellt (0,2 mg Amphotericin B/ml Emulsion), die Hochdruckhomogenisation der Emulsion erfolgte bei Raumtemperatur. Der Arzneistoff wurde in 1,2%iger wäßriger Tween 80-Lösung angerieben, die Suspension vorhomogenisiert und 80 mg dieser Suspension mit 40g Lipofundin N 20% gemischt. Es wurden folgende Durchmesser gemessen: D50% 0,142 µm, D90% 0,282 µm, D95% 0,331 µm, D99% 0,459 µm und D100% 0,843 µm.

### Beispiel 7

Es wurde eine Emulsion analog Beispiel 6 hergestellt, die Amphotericin B-Konzentration betrug jedoch 1 mg/ml Emulsion. Es wurden folgende Durchmesser gemessen: D50% 0,245 µm, D90% 0,390 µm, D95% 0,426 µm, D99% 0,489 µm, D100% 0,700 µm.

### Beispiel 8

Es wurde eine Emulsion analog Beispiel 6 hergestellt, die Amphotericin B-Konzentration betrug jedoch 2 mg/ml Emulsion. Es wurden folgende Durchmesser gemessen: D50% 0,237 µm, D90% 0,389 µm, D95% 0, 426 µm, D99% 0,491 µm, D100% 0, 701 µm.

### Beispiel 9

Es wurde eine Emulsion analog Beispiel 6 hergestellt, die Hochdruckhomogenisation der Emulsion erfolgte bei 60°C. Es wurden folgende Durchmesser gemessen: D50% 0,19.7 µm, D90% 0,388 µm, D95% 0,436 µm, D99% 0,532 µm und D100% 0,953 µm.

### Beispiel 10

Es wurde eine Emulsion analog Beispiel 7 hergestellt, der Homogenisationsdruck betrug jedoch 500 bar anstatt 1500 bar. Es wurden folgende Durchmesser gemessen: D50% 0,263 µm, D90% 0,401 µm, D95% 0,435 µm, D99% 0,493 µm und D100% 0,657 µm.

### Beispiel 11

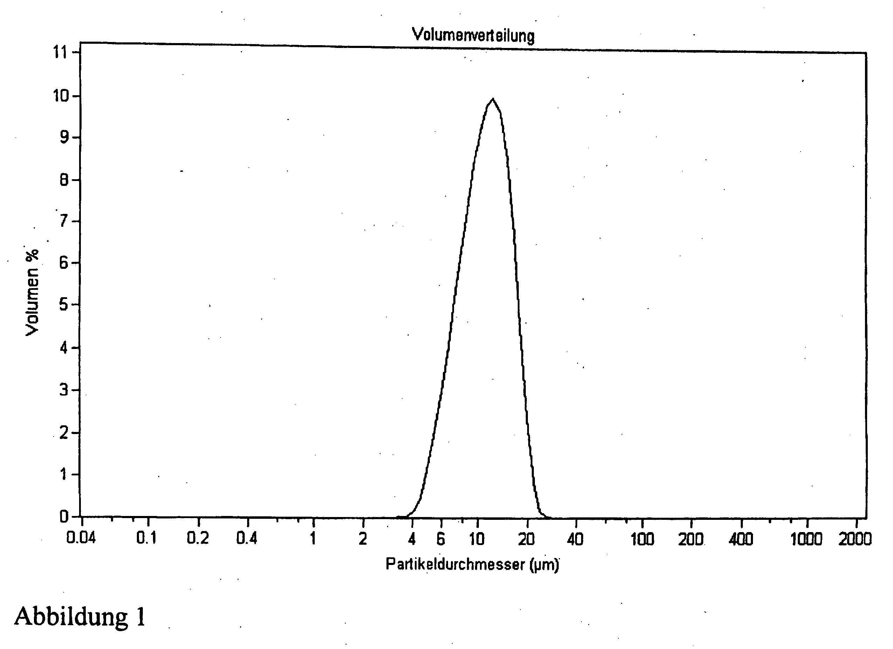

Die Partikelgrößenverteilung des Amphotericin B-Pulvers wurde mit Laserdiffraktometrie und Lichtmikroskopie analysiert. Abbildung 1 (oben) zeigt die Teilchengrößenverteilungskurve des Pulvers nach Dispergierung in Wasser ermittelt mit Laserdiffraktometrie sowie die Partikelgrößenverteilung nach Einarbeitung in das erfindungsgemäße Emulsionssystem aus Beispiel 2 (Abbildung 1, unten). Im Emulsionssystem sind keine Amphotericin B-Kristalle mehr detektierbar, Amphotericin B wurde in das Emulsionssystem inkorporiert.

### Beispiel 12

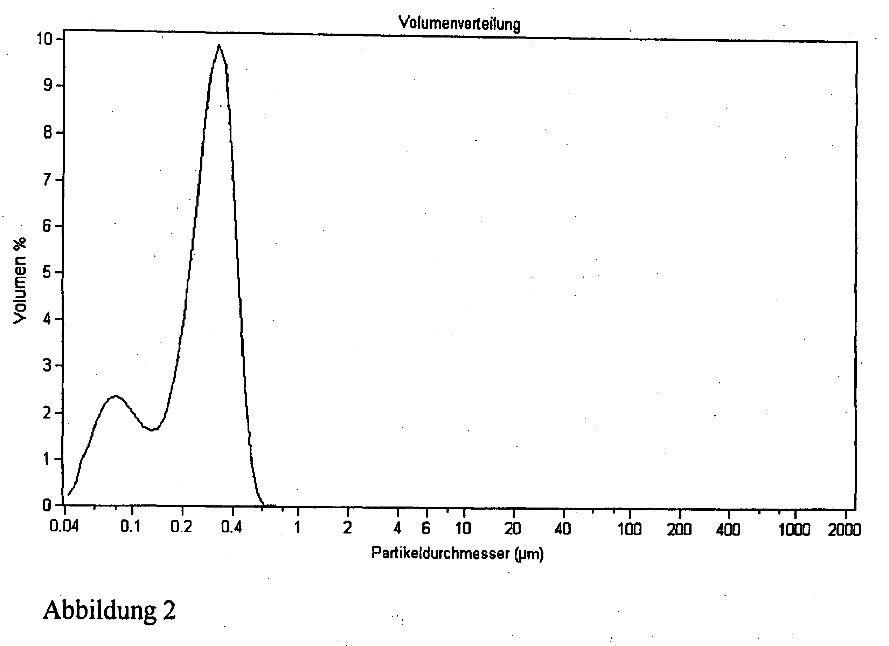

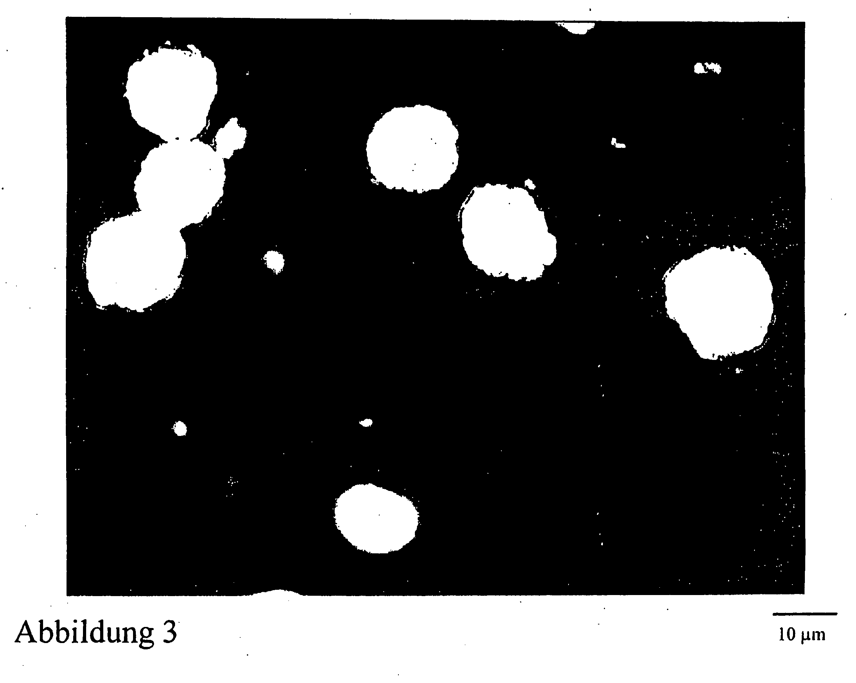

Die Amphotericin B-Emulsion wurde im Vergleich zu in Wasser dispergierten Amphotericin B-Kristallen mit Lichtmikroskopie untersucht. Abbildung 2 zeigt die lichtmikroskopische Aufnahme des Amphotericin B-Pulvers im polarisierten Licht, aufgrund der Anisotropie der Kristalle erscheinen sie hell. Abbildung 3 zeigt die lichtmikroskopische Aufnahme im polarisierten Licht nach Einarbeitung von Amphotericin B in das Emulsionssystem (Beispiel 1), anisotrope Strukturen sind nicht mehr detektierbar, das gesamte Bild ist nahezu schwarz. Für die Lichtmikroskopie wurde das Emulsionssystem unverdünnt auf den Objektträger aufgetragen.

### Beispiel 13

Buparvaquon wurde analog zu Amphotericin B wie in Beispiel 6 in ein Emulsionssystem eingearbeitet. Es wurden folgende Durchmesser gemessen: D50% 0,399 µm, D90% 0,527 µm, D95% 0,564 µm, D99% 0,635 µm und D100% 0,843 µm.

### Beispiel 14

Atovaquon wurde analog zu Beispiel 1 anstelle von Amphotericin B in ein Emulsionssystem eingearbeitet. Es wurden folgende Durchmesser gemessen: D50% 0,297 µm, D90% 0,437µm, D95% 0,475 µm, D99% 0,540 µm und D100% 0,744 µm.

### Beispiel 15

Es wurde eine Emulsion analog Beispiel 1 hergestellt, die Menge an eingearbeitetem Amphotericin betrug jedoch 5 mg/ml Emulsion. Die Löslichkeit in der Dispersion für Amphotericin war überschritten, neben Öltropfen lagen Arzneistoffkristalle vor (heterogene Dispersion).

### Beispiel 16

Es wurde eine Amphotericin B-Emulsion durch Zumischung von 40 mg Amphotericin B zu 40 ml Lipofundin N 20 % hergestellt (d. h. Amphotericin B 1 mg/ml Emulsion). Die Mischung wurde mit 10 Zyklen bei 1500 bar und 45°C homogenisiert. Diese Emulsion wurde durch Autoklavieren bei 121°C für 15 Minuten (gemäß Deutschen Arzneibuches) sterilisiert. Der PCS-Durchmesser vor Autoklavierung betrug 203 nm, der Polydispersitätsindex 0,102, nach Autoklavierung lag der Durchmesser bei 208 nm, der Polydispersitätsindex bei 0,137.

### Beispiel 17

100 mg Amphotericin B-Pulver wurden in 900 mg sterilen Wasser dispergiert, vorhomogenisiert und unter Verwendung von Pistill und Mörser in 20 g MCT-Öl mit 1,2% Lecithin eingearbeitet. Das Öl wurde in 80 g Wasser dispergiert und diese Mischung in einem Microfluidizer Typ Microfluidix M110y homogenisiert (d. h. Amphotericin B 1 mg/ml Emulsion). Die Homogenisation wurde bei 1000 bar für 10 Minuten durchgeführt. Der PCS-Durchmesser vor Autoklavierung betrug 192 nm, der Polydispersitätsindex 0,113, nach Autoklavierung lag der Durchmesser bei 196 nm, der Polydispersitätsindex bei 0,109.

### Beispiel 18

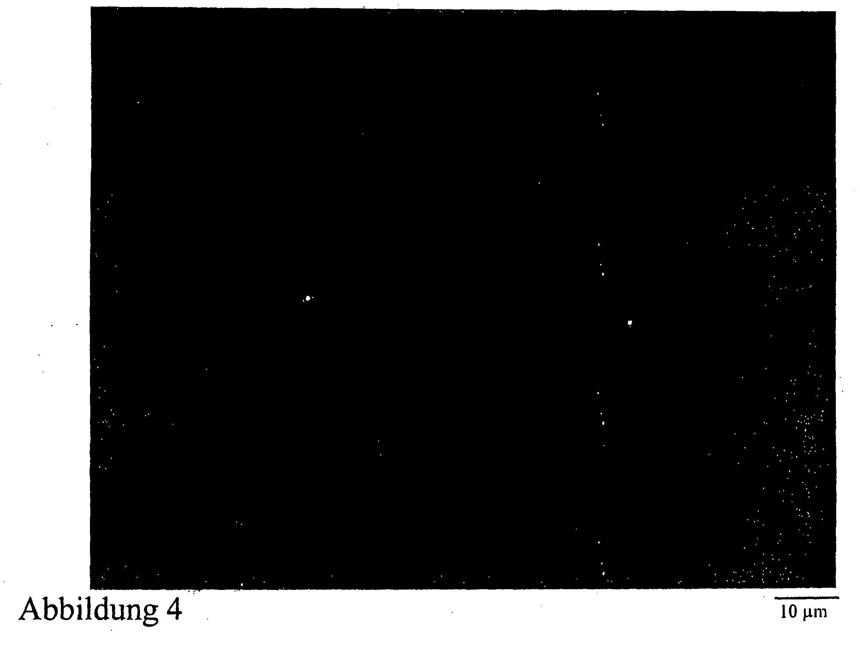

Die unverdünnte Amphotericin B-Emulsion aus Beispiel 17 wurde auf größere Partikel und Amphotericin B-Kristalle mittels Lichtmikroskop untersucht. Abbildung 4 zeigt nur wenige größere Tröpfchen, Amphotericin B-Kristalle konnten nicht detektiert werden.

### Beispiel 19

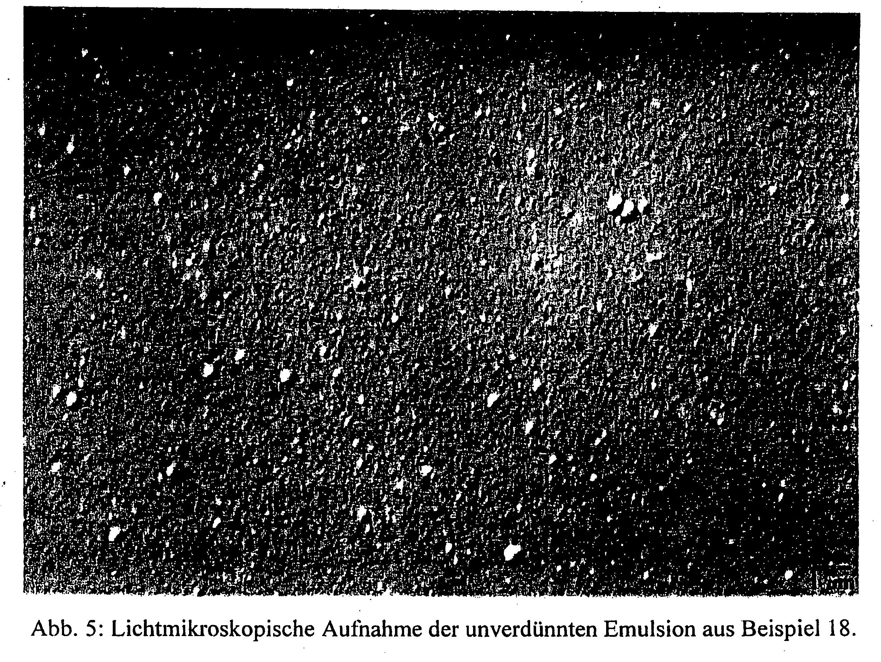

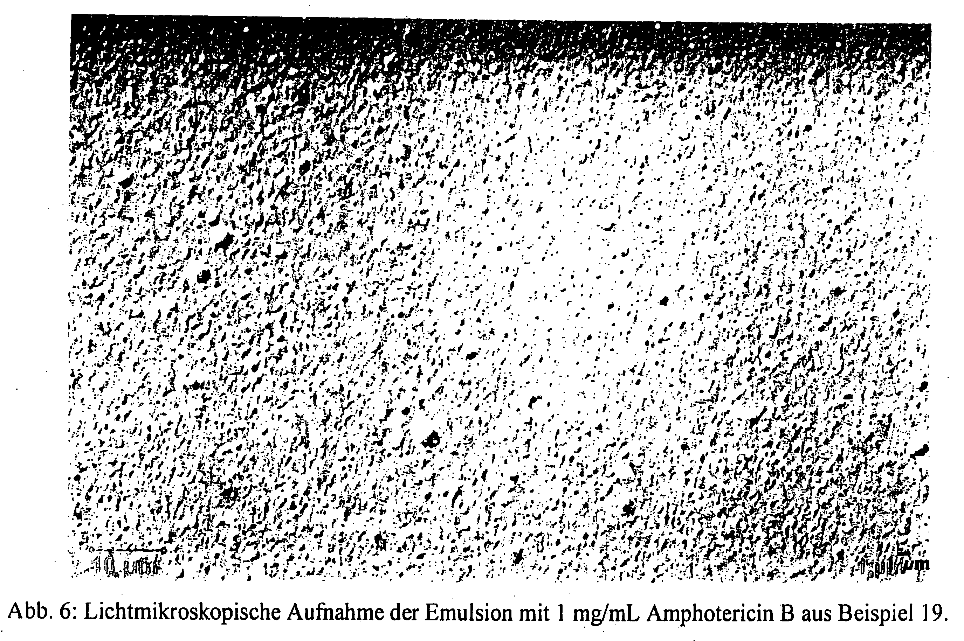

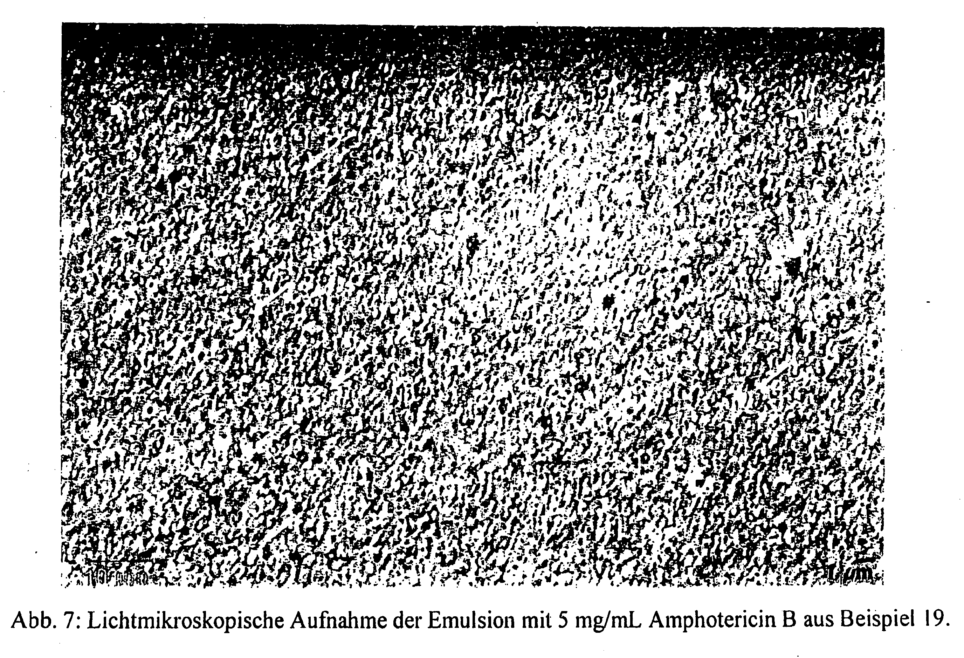

Es wurden Emulsionen, wie in Beispiel 16 beschrieben, hergestellt, wobei jedoch 15 Homogenisationszyklen durchgeführt wurden. Es wurden zwei Dispersionen hergestellt, die 1 mg/ml und 5 mg/ml Amphotericin B enthielten. Die Emulsionen wurden mit Lichtmikroskopie untersucht. Die lichtmikroskopische Aufnahme der Dispersion mit 1 mg/ml zeigt ein Emulsionssystem ohne detektierbare Amphotericin B-Partikel (Abb. 5), in der Dispersion mit 5 mg/ml Amphotericin B sind neben den Emulsionströpfchen kleine Amphotericin B-Kristalle detektierbar (Abb. 6)

### Beispiel 20

Es wurde eine Amphotericin B-Emulsion, wie in Beispiel 16, hergestellt. Die Emulsion wurde 20 Zyklen bei einer Produktionstemperatur von 65°C homogenisiert. Der mittlere PCS-Durchmesser betrug 255 nm, der Polydispersitätsindex 0,098. Die Partikelgröße wurde mittels Laserdiffraktometrie mit einem Coulter LS 230 (Coulter Electronics, USA) durchgeführt. Der Durchmesser 50% war 0,247 µm, der Durchmesser 90% 0,410 µm, der Durchmesser 99% 0,566 µm und der Durchmesser 100% 0,938 µm. Die Amphotericin B-Konzentration lag bei 1 mg/ml, Sterilisation wurde mittels Autoklavieren bei 121°C für 15 Minuten durchgeführt. Die Arzneistoffkonzentration wurde mit HPLC analysiert, wobei in zwei Proben 93,8% und 91,0% wiedergefunden wurden.

### Beispiel 21

100 mg Cyclosporin wurden mit 40 g Lipofundin N 20% angerieben. Die Homogenisation wurde mit 20 Zyklen bei 1500 bar und 25°C durchgeführt. Der mittlere PCS-Durchmesser betrug 234 nm, der Polydispersitätsindex 0,099. Der Laserdiffraktometerdurchmesser D50% lag bei 0,218 µm, der D90% bei 0,381 µm und der D100% bei 0,721 µm. Mit Lichtmikroskopie konnten keine Cyclosporin-Partikel detektiert werden (polarisiertes Licht, Dunkelfeld). Das Zetapotential der Emulsion wurde in destillierten Wasser mit einer eingestellten Leitfähigkeit von 50 µS/cm (durch Zugabe von Natriumchlorid) gemessen. Die Feldstärke lag bei 20 V/cm, die Umrechnung der elektrophoretischen Mobilität in das Zetapotential erfolgte mit der Helmholtz-Smoluchowski Gleichung. Das Zetapotential betrug -51 mV.

### Beispiel 22

Es wurde eine Cyclosporin-Emulsion wie in Beispiel 21 beschrieben hergestellt. Während der Produktion wurden jedoch 0,5% Cetylpyridiniumchlorid (CPC) zugefügt. Die Emulsion war positiv geladen, das Zetapotential betrug +32 mV.

### Beispiel 23

Es wurde eine Cyclosporin-Emulsion, wie in Beispiel 21 beschrieben, hergestellt. Während der Produktion wurden jedoch 1,0% Stearylamin zugefügt. Der PCS-Durchmesser betrug 247 nm, der Polydispersitätsindex 0.088. Der Laserdiffraktometerdurchmesser 50% lag bei 0,229 µm, der Durchmesser 90% bei 0,389 µm und der Durchmesser 100% bei 0,721 µm das Zetapotential betrug +24 mV.

### Beispiel 24

Eine Cyclosporin-Emulsion wurde de novo hergestellt. Die Zusammensetzung bestand aus 0,1% Cyclosporin, 0,5% Poloxamer 188, 0,5% Eilecithin Lipoid E80, 0,15% Stearylamin, 10% Miglyol 812 und 2,25% Glycerol als Isotonisierungszusatz und Wasser ad 100%. Das Lecithin wurde in der Öl-Phase dispergiert, eine Prä-Emulsion wurde unter Zusatz der anderen Bestandteile durch Hochgeschwindigkeitsrühren hergestellt, das Cyclosporin-Pulver wurde im letzten Schritt zugefügt. Diese Mischung wurde bei 45°C mit 20 Zyklen und 1500 bar homogenisiert. Der PCS-Durchmesser betrug 226 nm, der Polydispersitätsindex 0,111. Der Laserdiffraktometerdurchmesser 50% lag bei 0,200 µm, der Durchmesser 90% bei 0,406 µm und der Durchmesser 100% bei 1,154 µm. Die Emulsion war positiv geladen, das Zetapotential betrug +31 mV.

### Beispiel 25

Eine O/W-Dispersion wurde produziert mit der Zusammensetzung von 10 g Wasserphase, die 25 mg Amphotericin enthielt, 0,5 g Span 85, 0,25 Tween 80 und Miglyol 812 ad 50 g. 1,0 ml Amphotericin Suspension (2,5% Amphotericin/ml), stabilisiert mit 2,4% Lecithin Lipoid E 80 wurden gemischt mit destilliertem Wasser auf ein Gesamtgewicht von 10 g. Tween 80 wurde zur Wasserphase hinzugefügt, Span 85 zur Ölphase. Das Wasser wurde im Öl durch hochtouriges Rühren dispergiert. Die erhaltene Prä-Emulsion wurde bei 90°C homogenisiert unter Anwendung von 1500 bar und 20 Homogenisationszyklen. Größenanalytik wurde durchgeführt mit Laserdiffraktometrie (Mastersizer E, Malvern Instruments, United Kingdom). Der Durchmesser 50% war 2,25 µm, der Durchmesser 90% 4,21 µm.

### Erklärungen zu Abbildungen:

- Abb. 1:: Partikelgrößenverteilung des Amphotericin-Pulvers vor Einarbeitung in die Dispersion (oben) und Partikelgrößenanalyse der erfindungsgemäßen Dispersion nach Einarbeitung des Amphotericin-Pulvers (unten, Beispiel 2), die Arzneistoffpartikel sind nicht mehr detektierbar (Laserdiffraktometrie)
- Abb. 2:: Lichtmikroskopische Aufnahme des Amphotericin-Pulvers vor Einarbeitung in die O/W-Emulsion (Beispiel 1) (Polarisations-Aufnahme im Dunkelfeld, anisotrope Kristalle erscheinen weiß, Balken wie in Abb. 3 (10 µm)).
- Abb. 3:: Lichtmikroskopische Aufnahme der O/W-Emulsion nach Einarbeitung des Amphotericin-Pulvers aus Abb. 2 (Beispiel 1) (Polarisations-Aufnahme; im Dunkelfeld nur schemenhafte Reflexe der isotropen Emulsionstropfen, Balken 10 µm).
- Abb. 4:: Lichtmikroskopische Aufnahme der unverdünnten Emulsion aus Beispiel 18.
- Abb. 5:: Lichtmikroskopische Aufnahme der Emulsion mit 1 mg/ml AmphotericinB aus Beispiel 19.
- Abb. 6:: Lichtmikroskopische Aufnahme der Emulsion mit 5 mg/ml Amphotericin B aus Beispiel 19.

## Patentansprüche

1. Verfahren zur Herstellung einer Dispersion, die eine ölige Phase und eine wäßrige Phase in Form einer O/W-Emulsion oder einer Wasser-in-Öl (W/O) Emulsion, mindestens einen in der öligen und der wäßrigen Phase wenig oder schwer löslichen Wirkstoff sowie gegebenenfalls einen oder mehrere Emulgator(en) und/oder Stabilisator(en) umfaßt, wobei die Dispersion frei von toxikologisch bedenklichen organischen Lösungsmitteln ist und den Wirkstoff gelöst in einer Menge enthält, die höher ist als die Menge, die sich additiv aus seiner maximalen Löslichkeit in der öligen und der wäßrigen Phase der Emulsion ergibt, **dadurch gekennzeichnet**, eine wäßrige Phase und eine ölige Phase, die nicht oder nur teilweise miteinander mischbar sind, sowie gegebenenfalls ein oder mehrere Emulgator(en) und/oder Stabilisator(en) und eine feste Phase, die mindestens einen in der öligen und der wäßrigen Phase wenig oder schwer löslichen Wirkstoff umfaßt, miteinander gemischt werden und die erhaltene Mischung aus flüssigen und festen Phasen einem hochenergetischen Homogenisationsprozeß mit einem Homogenisator unterzogen werden, wobei keine toxikologisch bedenklichen organischen Lösungsmittel verwendet werden, wobei
a) der Wirkstoff ohne vorherige Auflösung als Feststoff in die flüssigen Phasen der Dispersion eingearbeitet wird,
b) der pulverisierte Wirkstoff mit einer O/W-Emulsion oder einer W/O-Emulsion angerieben oder gemischt wird und diese Prä-Dispersion der Homogenisation oder Hochdruckhomogenisation unterzogen wird oder
c) der pulverisierte wirkstoff in einer Emulgatorlösung dispergiert wird, diese Dispersion homogenisiert wird, anschließend mit einer O/W-Emulsion oder einer W/O-Emulsion gemischt wird und die so erhaltene Prä-Dispersion der Homogenisation oder Hochdruckhomogenisation unterzogen wird.

2. Verfahren nach Anspruch 1, **dadurch gekennzeichnet, daß**. der Wirkstoff ohne vorherige Auflösung als Feststoff in die flüssigen Phasen der Dispersion eingearbeitet wurde.

3. Verfahren nach Anspruch 1 oder 2, **dadurch gekennzeichnet, daß** der pulverisierte Wirkstoff mit einer O/W-Emulsion oder einer W/O-Emulsion angerieben oder gemischt wird und diese Prä-Dispersion der Homogenisation oder Hochdruckhomogenisation unterzogen wird.

4. Verfahren nach Anspruch 1 oder 2, **dadurch gekennzeichnet, daß** der pulverisierte Wirkstoff in einer Emulgatorlösung dispergiert wird, diese Dispersion homogenisiert wird, anschließend mit einer O/W-Emulsion oder einer W/O-Emulsion gemischt wird und die so erhaltene Prä-Dispersion der Homogenisation oder Hochdruckhomogenisation unterzogen wird.

5. Verfahren nach einem der Ansprüche 1 bis 4, **dadurch gekennzeichnet, daß** als Homogenisator ein Rotor-Stator-Homogenisator (vorzugsweise eine Kolloidmühle) oder ein Hochdruckhomogenisator (vorzugsweise ein Kolben-Spalt-Homogenisator (APV Gaulin, French Press, Niro, Stänsted) oder ein Rohrhomogenisator (jet stream) (Microfluidizer oder Nanojet)) eingesetzt wird.

6. Verfahren nach einem der Ansprüche 1 bis 5, **dadurch gekennzeichnet, daß** der Wirkstoff in einer solchen Menge eingesetzt wird, daß sich der Wirkstoff am Ende des Homogenisationsprozesses vollständig oder nahezu vollständig aufgelöst hat, so daß sich im Lichtmikroskop selbst bei 1000 facher Vergrößerung in 2 von 3 Feldern nicht mehr als 10 Kristalle, vorzugsweise nicht mehr als 5 Kristalle und insbesondere nicht mehr als 1 Kristall nachweisen lassen/läßt.

7. Verfahren nach einem der Ansprüche 1 bis 5, **dadurch gekennzeichnet, daß** der Wirkstoff in einer solchen Menge eingesetzt wird, daß am Ende des Homogenisationsprozesses neben dem gelösten Anteil des Wirkstoffs noch ein Anteil des Wirkstoffs in ungelöster kristalliner Form vorliegt, der ein Depot bildet.

8. Verfahren nach einem der Ansprüche 1 bis 7, **dadurch gekennzeichnet, daß** die Partikel des Wirkstoffes in ungelöster kristalliner Form einen Durchmesser 90% kleiner als 5 µm, bevorzugt einen Durchmesser 95% kleiner als 5 µm und insbesondere einen Durchmesser 100% kleiner als 5µm besitzen (Volumenverteilung bestimmt mit Laserdiffraktometrie).

9. Verfahren nach Anspruch 8, **dadurch gekennzeichnet, daß** die Partikel des Wirkstoffes in ungelöster kristalliner Form einen Durchmesser 90% kleiner als 3 µm, bevorzugt einen Durchmesser 95% kleiner als 3 µm und insbesondere einen Durchmesser 100% kleiner als 3µm besitzen (Volumenverteilung bestimmt mit Laserdiffraktometrie).

10. Verfahren nach Anspruch 9, **dadurch gekennzeichnet, daß** die Partikel des Wirkstoffes in ungelöster kristalliner Form einen mit Photonenkorrelationsspektroskopie (PCS) bestimmten Durchmesser kleiner als 1000 nm aufweisen.

11. Verfahren nach einem der Ansprüche 1 bis 10, **dadurch gekennzeichnet, daß** der Arzneistoff zusätzlich zum gelösten Zustand noch in hochdisperser fester kristalliner Form vorliegt, wodurch sich eine Dispersion mir einer heterogenen dispersen Phase aus Öltropfen und aus Arzneistoffkristallen ergibt.

12. Verfahren nach einem der Ansprüche 1 bis 11, **dadurch gekennzeichnet, daß** die Dispersion eine Öl-in-Wasser-Emulsion ist und, bezogen auf die Gesamtmenge der Dispersion, 5 bis 99,5 Gew.-%, vorzugsweise 10 bis 95 Gew.-% insbesondere 60 bis 95 Gew.-% und speziell 70-95% wässrige Phase enthält.

13. Verfahren nach einem der Ansprüche 1 bis 11, **dadurch gekennzeichnet, daß** die Dispersion eine wasser-in-Öl (W/O) Emulsion ist und, bezogen auf die Gesamtmenge der Dispersion, 5 bis 30 Gew.-%, vorzugsweise 10 bis 25 Gew.-% insbesondere 10 bis 20 Gew.-% wässrige Phase enthält.

14. Verfahren nach einem der vorhergehenden Ansprüche, **dadurch gekennzeichnet, daß** die Dispersion Emulgator und/oder Stabilisator enthält.

15. Verfahren nach Anspruch 14, **dadurch gekennzeichnet, daß** die Dispersion, bezogen auf die Gesamtmenge Dispersion, weniger als 15%, bevorzugt weniger als 10% und insbesondere weniger als 2%, bevorzugt 0,6% bis 1,2% Emulgator und/oder Stabilisator enthält.

16. Verfahren nach einem der Ansprüche 1 bis 15, **dadurch gekennzeichnet, daß** die Dispersion als Emulgatoren Ei-Lecithin, Soja-Lecithin, Phospholipide aus Ei oder Soja, Sorbitanestern (insbesondere Span 85), Polyethylenglycolsorbitanester (insbesondere Tween 80), Natriumglycocholat, Natriumlaurylsulfat (SDS) oder Gemischen derselben und/oder als Stabilisatoren Block-Copolymere, insbesondere Poloxamere (bevorzugt Poloxamer 188 und 407) oder Poloxamine (bevorzugt Poloxamine 908), Polyvinylpyrrolidon (PVP), Polyvinylalkohol (PVA), Gelatine, Polysaccharide (bevorzugt Hyaluronsäure oder Chitosan und ihre Derivate), Polyacrylsäure und ihre Derivate, Polycarbophil, Cellulosederivate (bevorzugt Methyl-, Hydroxypropyl- und Carboxymethylcellulose), Zuckerester (bevorzugt Saccharosemonostearat) oder Natriumcitrat einzeln oder in irgendeiner Mischung derselben enthält.

17. Verfahren nach einem der vorhergehenden Ansprüche, **dadurch gekennzeichnet, daß** die Dispersion eine O/W-Emulsion umfaßt und die zur Herstellung der Dispersion verwendete ölige Phase (Lipidphase) nur bei Raumtemperatur feste Lipide oder nur bei Raumtemperatur flüssige Lipide umfaßt oder eine Mischung aus einem oder mehreren bei Raumtemperatur flüssigen Lipiden mit einem oder mehreren bei Raumtemperatur festen Lipiden umfaßt.

18. Verfahren nach Anspruch 17, **dadurch gekennzeichnet, daß** die Mischung aus flüssigem Lipid und festem Lipid von 99 + 1 bis zu 1 + 99 variiert (Gewichtsteile), insbesondere in der Mischung der Anteil von flüssigem Lipid mindestens 10 Teile beträgt, bevorzugt mindestens 30 Teile und insbesondere mindestens 50 Teile.

19. Verfahren nach einem der vorhergehenden Ansprüche, **dadurch gekennzeichnet, daß** die Partikel aus folgenden einzelnen Lipiden oder deren Mischungen hergestellt werden: natürliche oder synthetische Triglyceride bzw. Mischungen derselben, Monoglyceride und Diglyceride, alleine oder Mischungen derselben oder mit Triglyceriden, selbstemulgierende modifizierte Lipide, natürliche und synthetische Wachse, Fettalkohole, einschließlich ihrer Ester und Ether und Mischungen derselben insbsondere synthetische Monoglyceride, Diglyceride und Triglyceride als individuelle Substanzen oder als Mischung, vorzugsweise Hartfett, oder Imwitor 900, Triglyceride, insbeondere Glyceroltrilaurat, Glycerolmyristat, Glycerolpalmitat, Glycerolstearat und Glycerolbehenat, und Wachse, insbesondere Cetylpalmitat, Carnaubawachs und weißes Wachs (DAB, sowie Kohlenwasserstoffe, insbeosndere Hartparaffin.

20. Verfahren nach einem der vorhergehenden Ansprüche, **dadurch gekennzeichnet, daß** die Dispersion als Ölphase Sojaöl, Safloröl, langkettige Triglyceride (LCT), mittelkettige Triglyceride (MCT), insbesondere Miglyole, Fischöle und Öle mit einem erhöhten Anteil an ungesättigten Fettsäuren, acetylierte Partialglyceride (bevorzugt wie in Stesolid) einzeln oder in Mischungen enthält.

21. Verfahren nach einem der vorhergehenden Ansprüche, **dadurch gekennzeichnet, daß** die Dispersion als wäßrige Phase Wasser, Mischungen von Wasser mit wassermischbaren organische Flüssigkeiten, insbesondere flüssigen Polyethylenglykolen (PEG) (bevorzugt PEG 400 und 600) enthält.

22. Verfahren nach einem der vorhergehenden Ansprüche, **dadurch gekennzeichnet, daß** die wäßrige Phase Zusätze enthält, insbesondere Elektrolyte, Nichtelektrolyte (bevorzugt Glycerol, Glucose, Mannit, Xylit zur Isotonisierung) und/oder Gelbildner (bevorzugt Cellulosederivate zur Viskositätserhöhung).

23. Verfahren nach einem der vorhergehenden Ansprüche, **dadurch gekennzeichnet, daß** die eingesetzte Emulsion eine O/W-Emulsion ist und Lipofundin, Intralipid, Lipovenös, Abbolipid, Deltalipid oder Salvilipid ist.

24. Verfahren nach einem der vorhergehenden Ansprüche, **dadurch gekennzeichnet, daß** der Wirkstoff ausgewählt ist aus der Gruppe bestehend aus Arzneiwirkstoffen zur Behandlung des menschlichen und tierischen Körpers.

25. Verfahren nach Anspruch 24, **dadurch gekennzeichnet, daß** die Dispersion einen oder mehrere Arzneistoffe aus den Gruppen der Anaesthetika, Antibiotika, Antimykotika, Antiinfektiva, Kortikoide, Hormone, Antioestrogene, Antispetika, gefäßaktive Sub-stanzen, Glaukomittel, Beta-Blocker, Cholinergika, Sympathomimetika, Carboanhydrase-Hemmer, Mydriatika, Virustatika, Mittel zur Tumortherapie, Antiallergika, Vitamine, antiinflammatorische Wirkstoffe sowie Immunsuppressiva enthalten, insbesondere Cyclosporin, oder irgendeine Kombination daraus enthält.

26. Verfahren nach einem der vorhergehenden Ansprüche, **dadurch gekennzeichnet, daß** die Dispersion positiv geladen ist.

27. Verfahren nach einem der vorhergehenden Ansprüche, **dadurch gekennzeichnet, daß** die Dispersion positiv geladene Stabilisatoren enthält, insbesondere Natriumlaurylsulfat (SDS), Stearylamin, und/oder positiv geladene Phospholipide und/oder positiv geladene Lipide.

28. Verfahren nach Anspruch 27, **dadurch gekennzeichnet, daß** die eingesetzte Emulsion eine O/W-Emulsion ist und intravenös appliziert werden kann, wobei neben positiven Stabilitsatoren auch Mischungen mit Lecithin und/oder nichtionischen Stabilisatoren eingesetzt werden können, insbesondere Poloxamer Polymere.

29. Verfahren nach einem der vorhergehenden Ansprüche, **dadurch gekennzeichnet, daß** die Dispersion Cyclosporin als Wirkstoff enthält.

30. Verfahren nach einem der Ansprüche 1 bis 29, **dadurch gekennzeichnet, daß** die Dispersion als Wirkstoff ein Antimykotikum (bevorzugt Amphotericin B), ein Antiinfektivum (bevorzugt Buparvaquon oder Atovaquon), ein Immunsuppressivum (bevorzugt Cyclosporin A oder eines seiner natürlichen und synthetischen Derivate), ein Mittel zur Tumortherapie (bevorzugt Paclitaxel oder Taxotere) enthält.

## Claims

1. Method for the production of a dispersion which comprises an oily phase and an aqueous phase in the form of an O/W emulsion or an W/O emulsion, at least one active ingredient that is only slightly or with difficulty soluble in the oily and the aqueous phase, and optionally one or more emulsifier(s) and/or stabilizer(s), wherein the dispersion is free from toxicologically dangerous organic solvents and contains the active ingredient dissolved in a quantity that is greater than the quantity which results additively from its maximum solubility in the oily and the aqueous phase of the emulsion, **characterized in that** an aqueous phase and an oily phase, which are not, or are only partially miscible with one another, and optionally one or more emulsifier(s) and/or stabilizer(s) and a solid phase, which comprises at least one active ingredient which can be dissolved in the oily and the aqueous phase only slightly or with difficulty, are mixed together, and the mixture of liquid and solid phases obtained is subjected to a high-energy homogenization process with a homogenizer without using toxicologically dangerous organic solvents, wherein
(a) the active ingredient is incorporated as a solid into the liquid phases of the dispersion without previously being dissolved,
(b) the pulverized active ingredient is triturated or mixed with an O/W emulsion or an W/O emulsion and this pre-dispersion is subjected to the homogenization or high pressure homogenization process, or
(c) the pulverized active ingredient is dispersed in a surfactant solution, this dispersion is homogenized, then mixed with an O/W emulsion or an W/O emulsion and the pre-dispersion thus obtained is subjected to the homogenization or high pressure homogenization process.

2. Method according to Claim 1, **characterized in that** the active ingredient is incorporated as a solid into the liquid phases of the dispersion without previously being dissolved.

3. Method according to Claim 1 or 2, **characterized in that** the pulverized active ingredient is triturated or mixed with an O/W emulsion or an W/O emulsion and this pre-dispersion is subjected to the homogenization or high pressure homogenization process.

4. Method according to Claim 1 or 2, **characterized in that** the pulverized active ingredient is dispersed in a surfactant solution, this dispersion is homogenized, then mixed with an O/W emulsion or an W/O emulsion and the pre-dispersion thus obtained is subjected to the homogenization or high pressure homogenization process.

5. Method according to any of Claims 1 to 4, **characterized in that** a rotor-stator homogenizer (preferably a colloid mill) or a high pressure homogenizer (preferably a piston homogenizer (APV Gaulin, French Press, Niro, Stansted) or a tube homogenizer (jet stream) (Microfluidizer or Nanojet)) is used as homogenizer.

6. Method according to any of Claims 1 to 5, **characterized in that** the active ingredient is used in a quantity such that the active ingredient at the end of the homogenization process has dissolved completely, or almost completely, so that with a light microscope, even with 1000-fold magnification, in 2 out of 3 fields, no more than 10 crystals, preferably no more than 5 crystals and especially no more than 1 crystal can be detected.

7. Method according to any of Claims 1 to 5, **characterized in that** the active ingredient is used in a quantity such that, at the end of the homogenization process, besides the portion of the dissolved active ingredient, a portion of the active ingredient is still present in undissolved crystalline form, which forms a depot.

8. Method according to any of Claims 1 to 7, **characterized in that** the particles of the active ingredient in undissolved crystalline form have a diameter of 90%, prefereably 95% and in particular 100% smaller than 5 µm (volume distribution determined by laser diffractometry).

9. Method according to Claim 8, **characterized in that** the particles of the active ingredient in undissolved crystalline form have a diameter of 90%, prefereably 95% and in particular 100% smaller than 3 µm (volume distribution determined by laser diffractometry).

10. Method according to Claim 9, **characterized in that** the particles of the active ingredient in undissolved crystalline form have a photon correlation spectroscopy (PCS) diameter smaller than 1000 nm.

11. Method according to any of Claims 1 to 10, **characterized in that** the drug, in addition to the dissolved state, is still present in highly dispersed solid crystalline form, resulting in a dispersion with a heterogeneously dispersed phase of oil drops and drug crystals.

12. Method according to any of Claims 1 to 11, **characterized in that** the dispersion is an oil-in-water emulsion and, based on the total amount of the dispersion, contains 5 to 99.5 wt.%, preferably 10 to 95 wt.%, more preferred 60 to 95 wt.% and specifically 70 to 95 wt.% of aqueous phase.

13. Method according to any of Claims 1 to 11, **characterized in that** the dispersion is an water-in-oil (W/O) emulsion and, based on the total amount of the dispersion, contains 5 to 30 wt.%, preferably 10 to 25 wt.% and in paricular 10 to 20 wt.% of aqueous phase.

14. Method according to any of the preceeding Claims, **characterized in that** the dispersion contains emulsifier and/or stabilizer.

15. Method according to Claim 14, **characterized in that** the dispersion contains, based on the total amount of the dispersion, less than 15%, preferably less than 10% and in particular less than 2%, preferably 0.6 to 1.2% of emulsifier and/or stabilizer.

16. Method according to any of Claims 1 to 15, **characterized in that** the dispersion contains as emulsifiers egg lecithin, soya lecithin, phospholipids of egg or soya, sorbitan esters (in particular Span 85), polyethylene glycol sobitan esters (in particular Tween 80), sodium glycocholate, sodium lauryl sulphate (SDS) or mixtures thereof, and/or, as stabilizers, block co-polymers, especially poloxamers (preferably Poloxamer 188 and 407) or poloxamines (preferably Poloxamine 908), polyvinyl pyrrolidon (PVP), polyvinyl alcohol (PVA), gelatine, polysaccharide (preferably hyaluronic acid or chitosan and its derivatives), polyacryl acid and its derivatives, polycarbophil, cellulose derivatives (preferably methyl, hydroxypropyl and carboxymethyl cellulose), sugar esters (preferably saccharose monostearate) or sodium citrate individually or in any mixture thereof.

17. Method according to any of the preceeding Claims, **characterized in that** the dispersion comprises an O/W emulsion and the oil phase (lipid phase) used for the preparation of the dispersion comprises only lipids which are solid at room temperature or only lipids which are liquid at room temperature or a mixture of one or more lipids which are liquid at room temperature with one or more lipids which are solid at room temperature.

18. Method according to Claim 17, **characterized in that** the mixture of liquid lipid and solid lipid varies from 99 + 1 to 1 + 99 (parts by weight), in particular the proportion of liquid lipid in the mixture is at least 10 parts, preferably at least 30 parts and more preferred 50 parts.

19. Method according to any of the preceeding Claims, **characterized in that** the particles are prepared from the following individual lipids or mixtures thereof: natural or synthetic triglycerides or mixtures thereof, monoglycerides and diglycerides, individually or mixtures thereof or with triglycerides, self-emulsifying modified lipids, natural and synthetic waxes, fatty alcohols, including their esters and ethers and mixtures thereof, in particular synthetic monoglycerides, diglycerides and triglycerides as individual substances or as mixture, pre-ferably hard wax, or Imwitor 900, triglycerides, in particular glycerol trilaurate, glycerol myristate, glycerol palmitate glycerol stearate and glycerol behenat, and waxes, in particular cetyl palmitate, carnauba wax and white wax (German pharma-copeia DAB), as well as hydrocarbons, in particular hard paraffin.

20. Method according to any of the preceeding Claims, **characterized in that** the dispersion Method according to any of the preceeding Claims, **characterized in that** the dispersion contains, as an oil phase, soya oil, safflower oil, long-chain triglycerides (LCT), medium-chain triglycerides (MCT) especially miglyols, fish oils and oils with an increased constituent of unsaturated fatty acids, acetylated partial glycerides (preferably as in Stesolid) individually or in mixtures.

21. Method according to any of the preceeding Claims, **characterized in that** the dispersion contains, as an aqueous phase, water, mixtures of water with water-miscible organic liquids, especially liquid polyethylene glycols (PEC) (preferably PEG 400 and 600).

22. Dispersion according to any of the preceeding Claims, **characterized in that** the aqueous phase contains additives, especially electrolytes, non-electrolytes (preferably glycerol, glucose, mannitol, xylite for isotonization) and/or gel forming agents (preferably cellulose derivatives to increase viscosity).

23. Method according to any of the preceeding Claims, **characterized in that** the emulsion used is a O/W emulsion and is Lipofundin, Intralipid, Lipovenoes, Abbolipid, Deltalipid or Salvilipid.

24. Method according to any of the preceeding Claims, **characterized in that** the active ingredient is selected from the group consisting of medical drugs for treatment of human or animal bodies.

25. Method according to Claim 24, **characterized in that** the dispersion contains one or more active ingredient(s) seleted from the group consisting of aenesthetics, antibiotics, antimycotics, antiinfectives, corticoids, hormons, antiestrogens antiseptics, vasoactivating agents, glauco agents, beta blocker, cholinergics, sympathomimetics, carboanhydrase inhibitors, mydriatics, virustatics, agents for tumor therapy, antiallergics, vitamins, antiinflammytory drugs, as well as immuno-supressives, in particular ciclosporine, or any combination thereof.

26. Method according to any of the preceeding Claims, **characterized in that** the dispersion is positively charged.

27. Method according to any of the preceeding Claims, **characterized in that** the dispersion contains positively charged stabilzers, in particular sodium lauryl sulfate (SDS), stearylamine, and/or positively charged phospholipids and/or positively charged lipids.

28. Method according to Claim 27, **characterized in that** the emulsion used is a O/W emulsion and can be applied intraveneously, wherein in addition to positively charged stabilizers also mixtures with lecithines and/or nonionic stabilizers may be used, in particular poloxamer polymers.

29. Method according to any of the preceeding Claims, **characterized in that** the dispersion contains ciclosporine as active ingredient.

30. Method according to any of Claims 1 to 29, **characterized in that** the dispersion contains, as active ingredient, an antimycotic (preferably Amphotericin B), an antiinfective (preferably Buparvaquone or Atovaquone), an immunosuppressive (preferably Cyclosporin A or one of its natural and synthetic derivatives), a tumour therapy drug (preferably Paclitaxel or Taxotere).

## Revendications

1. Procédé pour la préparation d'une dispersion qui comprend une phase huileuse et une phase aqueuse sous forme d'une émulsion H/E ou d'une émulsion eau-dans-huile (E/H), au moins une substance active peu ou difficilement soluble dans la phase huileuse et dans la phase aqueuse ainsi qu'éventuellement un ou plusieurs émulsifiant(s) et/ou stabilisant(s), la dispersion étant exempte de solvants organiques suspects du point de vue toxicologique et contenant la substance active dissoute en une quantité qui est supérieure à la quantité qui résulte de l'addition de sa solubilité maximale dans la phase huileuse et dans la phase aqueuse de l'émulsion, **caractérisé en ce qu'**on mélange ensemble une phase aqueuse et une phase huileuse, qui ne sont pas miscibles ou ne sont que partiellement miscibles entre elles, ainsi qu'éventuellement un ou plusieurs émulsifiant(s) et/ou stabilisant(s) et une phase solide qui comprend au moins une substance active peu ou difficilement soluble dans la phase huileuse et dans la phase aqueuse, et on soumet le mélange obtenu de phases liquides et de phase solide à un processus d'homogénéisation à haute énergie, en n'utilisant pas de solvants organiques suspects du point de vue toxicologique,
a) la substance active étant incorporée sous forme solide, sans dissolution préliminaire, dans les phases liquides de la dispersion,
b) la substance active pulvérisée étant broyée ou mélangée avec une émulsion H/E ou une émulsion E/H et cette pré-dispersion étant soumise à l'homogénéisation ou à l'homogénéisation sous haute pression ou
c) la substance active pulvérisée étant dispersée dans une solution d'émulsifiant, cette dispersion étant homogénéisée, puis étant mélangée avec une émulsion H/E ou une émulsion E/H et la pré-dispersion ainsi obtenue étant soumise à l'homogénéisation ou à l'homogénéisation sous haute pression.

2. Procédé selon la revendication 1, **caractérisé en ce que** la substance active a été incorporée sous forme solide, dans dissolution préliminaire, dans les phases liquides de la dispersion.

3. Procédé selon la revendication 1 ou 2, **caractérisé en ce que** la substance active pulvérisée est broyée ou mélangée avec une émulsion H/E ou une émulsion E/H et cette pré-dispersion est soumise à l'homogénéisation ou à l'homogénéisation sous haute pression.

4. Procédé selon la revendication 1 ou 2, **caractérisé en ce que** la substance active pulvérisée est dispersée dans une solution d'émulsifiant, on homogénéise cette dispersion, ensuite on la mélange avec une émulsion H/E ou une émulsion E/H et on soumet la pré-dispersion ainsi obtenue à l'homogénéisation ou à l'homogénéisation sous haute pression.

5. Procédé selon l'une quelconque des revendications 1 à 4, **caractérisé en ce qu'**on utilise comme homogénéisateur un homogénéisateur à rotor-stator (de préférence un broyeur colloïdal) ou un homogénéisateur à haute pression (de préférence un homogénéisateur à piston-fente (APV Gaulin, presse de French, Niro, Stansted) ou un homogénéisateur tubulaire (jet stream) (Microfluidizer ou Nanojet).

6. Procédé selon l'une quelconque des revendications 1 à 5, **caractérisé en ce qu'**on utilise la substance active en une quantité telle que la substance active est totalement ou dissoute sensiblement en totalité à la fin du processus d'homogénéisation, de sorte qu'au microscope optique on ne peut déceler pas plus de 10 cristaux, de préférence pas plus de 5 cristaux et en particulier pas plus d'un cristal dans 2 champs sur 3, même à un grossissement de 1 000x.

7. Procédé selon l'une quelconque des revendications 1 à 5, **caractérisé en ce qu'**on utilise la substance active en une quantité telle qu'à la fin du processus d'homogénéisation, en plus de la fraction dissoute de la substance active, est encore présente une fraction de la substance active sous forme cristalline non dissoute, qui forme un dépôt.

8. Procédé selon l'une quelconque des revendications 1 à 7, **caractérisé en ce que** les particules de la substance active sous forme cristalline non dissoute on un diamètre à 90 % inférieur à 5 µm, de préférence un diamètre à 95 % inférieur à 5 µm et en particulier un diamètre à 100 % inférieur à 5 µm (distribution volumique déterminée par diffractométrie laser).

9. Procédé selon la revendication 8, **caractérisé en ce que** les particules de la substance active sous forme cristalline non dissoute on un diamètre à 90 % inférieur à 3 µm, de préférence un diamètre à 95 % inférieur à 3 µm, en particulier un diamètre à 100 % inférieur à 3 µm (distribution volumique déterminée par diffractométrie laser).

10. Procédé selon la revendication 9, **caractérisé en ce que** les particules de la substance active sous forme cristalline non dissoute ont un diamètre, déterminé par spectroscopie par corrélation de photons (PCS), inférieur à 1 000 nm.

11. Procédé selon l'une quelconque des revendications 1 à 10, **caractérisé en ce que** le principe actif en outre se trouve à l'état dissous encore sous forme solide cristalline hautement dispersée, de sorte qu'il en résulte une dispersion comportant une phase dispersée hétérogène composée de gouttes d'huile et de cristaux de principe actif.

12. Procédé selon l'une quelconque des revendications 1 à 11, **caractérisé en ce que** la dispersion est une émulsion huile-dans-eau et contient, par rapport à la quantité totale de la dispersion, de 5 à 99,5 % en poids, de préférence de 10 à 95 % en poids, en particulier de 60 à 95 % en poids et plus particulièrement de 70 à 95 % en poids de phase aqueuse.

13. Procédé selon l'une quelconque des revendications 1 à 11, **caractérisé en ce que** la dispersion est une dispersion eau-dans-huile (E/H) et contient, par rapport à la quantité totale de la dispersion, de 5 à 30 % en poids, de préférence de 10 à 25 % en poids, en particulier de 10 à 20 % en poids de phase aqueuse.

14. Procédé selon l'une quelconque des revendications précédentes, **caractérisé en ce que** la dispersion contient un émulsifiant et/ou un stabilisant.

15. Procédé selon la revendication 14, **caractérisé en ce que** la dispersion contient, par rapport à la quantité totale de dispersion, moins de 15 %, de préférence moins de 10 % et en particulier moins de 2 %, de préférence de 0,6 % à 1,2 % d'émulsifiant et/ou de stabilisant.

16. Procédé selon l'une quelconque des revendications 1 à 15, **caractérisé en ce que** la dispersion contient en tant qu'émulsifiants de la lécithine d'oeuf, de la lécithine de soja, des phospholipides d'oeuf ou de soja, des esters de sorbitanne (tel que le Span 85), des esters de sorbitanne et de polyéthylèneglycol (tel que le Tween 80), du glycocholate de sodium, du laurylsulfate de sodium (DSD) ou des mélanges de ceux-ci et/ou en tant que stabilisants des copolymères séquencés, en particulier des Poloxamers (de préférence Poloxamer 188 et 407) ou des Poloxamines (Poloxamine 908 de préférence), de la polyvinylpyrrolidone (PVP), du poly(alcool vinylique) (PVA), de la gélatine, des polysaccharides (de préférence de l'acide hyaluronique ou du chitosane et leurs dérivés), du poly(acide acrylique) et ses dérivés, du Polycarbophil, des dérivés de cellulose (de préférence méthyl-, hydroxypropyl- et carboxyméthylcellulose), des esters dérivés de sucres (de préférence du monostéarate de cellulose) ou du citrate de sodium, individuellement ou en un mélange quelconque.

17. Procédé selon l'une quelconque des revendications précédentes, **caractérisé en ce que** la dispersion comprend une émulsion H/E et la phase huileuse (phase lipidique) utilisée pour la préparation de la dispersion comprend uniquement des lipides solides à la température ambiante ou uniquement des lipides liquides à la température ambiante ou un mélange d'un ou plusieurs lipides liquides à la température ambiante avec un ou plusieurs lipides solides à la température ambiante.

18. Procédé selon la revendication 17, **caractérisé en ce que** le mélange de lipide liquide et de lipide solide varie de 99 + 1 à 1 + 99 (parties en poids), en particulier la proportion de lipide liquide dans le mélange est d'au moins 10 parties, de préférence d'au moins 30 parties et en particulier d'au moins 30 parties.

19. Procédé selon l'une quelconque des revendications précédentes, **caractérisé en ce que** les particules sont préparées à partir des lipides individuels suivants ou de mélanges de ceux-ci : des triglycérides naturels ou synthétiques ou leurs mélanges, des monoglycérides et diglycérides, seuls ou des mélanges de ceux-ci ou avec des triglycérides, des lipides modifiés auto-émulsifiants, des cires naturelles ou synthétiques, des alcools gras, y compris leurs esters et éthers et des mélanges de ceux-ci, en particulier des monoglycérides, diglycérides et triglycérides synthétiques en tant que substances individuelles ou sous forme de mélange, de préférence Adeps solidus ou Imwitor 900, des triglycérides, en particulier le trilaurate de glycérol, le myristate de glycérol, le palmitate de glycérol, le stéarate de glycérol et le béhénate de glycérol, et des cires, en particulier le palmitate de cétyle, la cire de carnauba et la cire blanche (DAB), ainsi que des hydrocarbures, notamment la paraffine dure.

20. Procédé selon l'une quelconque des revendications précédentes, **caractérisé en ce que** la dispersion contient en tant que phase huileuse de l'huile de soja, de l'huile de carthame, des triglycérides à longue chaîne (LCT), des triglycérides à longueur moyenne de chaîne (MCT), en particulier des Miglyols, des huiles de poisson et des huiles ayant une forte teneur en acides gras insaturés, des glycérides partiels acétylés (de préférence comme dans le Stesolid), individuellement ou en mélanges.

21. Procédé selon l'une quelconque des revendications précédentes, **caractérisé en ce que** la dispersion contient en tant que phase aqueuse de l'eau, des mélanges d'eau et de liquides organiques miscibles à l'eau, en particulier des polyéthylèneglycols (PEG) liquides (de préférence PEG 400 et 600).

22. Procédé selon l'une quelconque des revendications précédentes, **caractérisé en ce que** la phase aqueuse contient des additifs, en particulier des électrolytes, des non-électrolytes (de préférence du glycérol, du glucose, du mannitol, du xylitol pour l'ajustement de l'isotonie) et/ou des agents gélifiants (en particulier des dérivés de cellulose pour l'augmentation de la viscosité).

23. Procédé selon l'une quelconque des revendications précédentes, **caractérisé en ce que** l'émulsion utilisée est une émulsion H/E et est la Lipofundin, l'Intralipid, le Lipovenös, l'Abbolipid, le Deltalipid ou le Salvilipid.

24. Procédé selon l'une quelconque des revendications précédentes, **caractérisé en ce que** la substance active est choisie dans le groupe constitué par des principes actifs destinés au traitement de l'organisme humain ou animal.

25. Procédé selon la revendications précédente 24, **caractérisé en ce que** la dispersion contient un ou plusieurs principes actifs choisis dans les groupes des anesthésiques, antibiotiques, antimycosiques, anti-infectieux, corticoïdes, hormones, anti-oestrogènes, antiseptiques, substances vaso-actives, antiglocaumateux, bêta-bloquants, cholinergiques, sympathomimétiques, inhibiteurs de carboanhydrase, mydriatiques, virustatiques, agents pour la thérapie tumorale, antiallergiques, vitamines, substances actives anti-inflammatoires et immunosuppresseurs, en particulier de la cyclosporine, ou une quelconque association de ceux-ci.

26. Procédé selon l'une quelconque des revendications précédentes, **caractérisé en ce que** la dispersion est chargée positivement.

27. Procédé selon l'une quelconque des revendications précédentes, **caractérisé en ce que** la dispersion contient des stabilisants chargés positivement, en particulier du laurylsulfate de sodium (SDS), de la stéarylamine, et/ou des phospholipides chargés positivement et/ou des lipides chargés positivement.

28. Procédé selon la revendication 27, **caractérisé en ce que** l'émulsion utilisée est une émulsion H/E et peut être administrée par voie intraveineuse, outre des stabilisants chargés positivement, également des mélanges avec la lécithine et/ou des stabilisants non ioniques pouvant être utilisés, en particulier des polymères Poloxamer.

29. Procédé selon l'une quelconque des revendications précédentes, **caractérisé en ce que** la dispersion contient de la cyclosporine en tant que substance active.

30. Procédé selon l'une quelconque des revendications 1 à 29, **caractérisé en ce que** la dispersion contient en tant que substance active un antimycosique (l'amphotéricine B de préférence), un anti-infectieux (de préférence la buparvaquone ou l'atavaquone), un immunosuppresseur (de préférence la cyclosporine A ou l'un de ses dérivés naturels ou synthétiques), un agent destiné à la thérapie tumorale (de préférence le paclitaxel ou le Taxotere).
